(19) Europäisches Patentamt
European Patent Office
Office européen des brevets

(11) EP 1 672 989 B1

(12) EUROPEAN PATENT SPECIFICATION

(45) Date of publication and mention of the grant of the patent:
**25.04.2012 Bulletin 2012/17**

(21) Application number: **04783556.6**

(22) Date of filing: **08.09.2004**

(51) Int Cl.:
***A23L 3/3517*** *(2006.01)* ***A61L 2/00*** *(2006.01)*
***A61L 2/18*** *(2006.01)* ***A23B 4/20*** *(2006.01)*
***A23B 7/154*** *(2006.01)* ***A23L 3/3481*** *(2006.01)*
***A23L 3/349*** *(2006.01)*

(86) International application number:
**PCT/US2004/029344**

(87) International publication number:
**WO 2005/023023 (17.03.2005 Gazette 2005/11)**

(54) **CONCENTRATED ANTIMICROBIAL COMPOSITIONS AND METHODS**

KONZENTRIERTE ANTIMIKROBIELLE ZUSAMMENSETZUNGEN UND VERFAHREN

COMPOSITIONS ANTIMICROBIENNES CONCENTREES, ET PROCEDES

(84) Designated Contracting States:
**AT BE BG CH CY CZ DE DK EE ES FI FR GB GR HU IE IT LI LU MC NL PL PT RO SE SI SK TR**

(30) Priority: **09.09.2003 US 659584**

(43) Date of publication of application:
**28.06.2006 Bulletin 2006/26**

(60) Divisional application:
**10185545.0 / 2 308 326**

(73) Proprietor: **3M Innovative Properties Company
St. Paul, MN 55133-3427 (US)**

(72) Inventors:
- **ANDREWS, Jeffrey F.,
Saint Paul, MN 55133-3427 (US)**
- **WANG, Danli
Saint Paul, MN 55133-3427 (US)**

(74) Representative: **Elsy, David et al
Withers & Rogers LLP
4 More London Riverside
London SE1 2AU (GB)**

(56) References cited:
**EP-A- 0 876 768** **WO-A-01/43549**
**WO-A-95/07616** **US-A- 4 067 997**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 672 989 B1

## Description

BACKGROUND

The present invention is generally related to a composition and a kit to reduce the microbial contamination on organic matter, such as processed meat, fruits and vegetables, plant parts; and other inanimate surfaces such as textiles and stainless steel.

Food borne diseases cause significant illness and death each year, with direct and indirect medical costs estimated by some sources to be over 1 billion a year. Common food pathogens include *Salmonella, Listeria monocytogenes, Escherichia coli* 0157:H7, *Campylobacter jejuni, Bacillus cereus,* and Norwalk-like viruses. Outbreaks of food borne diseases typically have been associated with contaminated meat products, raw milk, or poultry products but fruits and vegetables can also serve as sources of food borne illness. Surface, containers and other substrates can be a source of contamination in food. Recalls of food products, such as ground beef, hot dogs, and alfalfa sprouts, and orange juice, show a need for a broad spectrum antimicrobial solution that is safe for humans, environmentally friendly and cost effective.

Compositions used to reduce the microbial contamination in and on food as well as other surfaces have typically involved use of materials such as organic acids and chlorine compounds, such as sodium hypochlorite, that at higher concentrations may affect the properties of the surface treated. Compositions using fatty acid monoesters have been used in recent years to reduce microbial load can food such as poultry as U.S. Patent Nos. 5,460,833 and 5,490,992, fruit and vegetables as described in publication WO 200143549A, and dried compositions used on textiles, U.S. Application Serial No. 09/572,549, filed May 17, 2000, and in contact lenses as described in U.S. Patent No. 4,485,029. The fatty acid monoesters in these compositions have limited stability in the presence of other components. The antimicrobial activity of the compositions is reduced over time through reactions such as transesterification or hydrolysis. Increased costs are also associated with shipment of these compositions due to the presence of high concentrations of a vehicle or carrier.

WO-A-95/07616 discloses a product-in-process to reduce the microbial contamination of processed meat and is particularly related to a product and a process to disinfect poultry carcasses using a disinfectant composition containing a fatty acid monoester, an acid or chelating agent, and a food grade surfactant. These components may be combined in either an aqueous or nonaqueous vehicle as desired. The present combination is effective against pathogenic and undesired bacteria. Advantageous, the present composition does not detrimentally alter the taste, texture, colour, odour or appearance of the processed meat.

WO 01/43549 A2 describes antimicrobial formulations that can be used to reduce levels of microbes on the surfaces plants.

US-A-4,067,997 discloses a microbecidal or microbiostatic water-dispersible synergistic combination of a non-ionic surface-active agent that is a mono-ester of a twelve carbon atom aliphatic acid and a polyol and at least one microbecide-to-ester are between 1:1 to 1:100. These compositions may be dispersed in water and avoid inactivation of the microbecide. Further, the present composition provides activity greater than either the ester or the microbecide alone.

EP-A-0 876 768 discloses a preserving method of food which has excellent preserving effect for food, is safe and does not exert undesirable influence on flavour and physical properties of food, and relates to a preserving method of food wherein a polyglycerin fatty acid monoester having a monoester content of 50% by weight or more is used in producing food, in particular, a fatty acid constituting the polyglycerin fatty acid monoester is composed on one or a mixture of two or more selected from caprylic acid, capric acid, lauric acid or myristic acid and the total amount thereof is 70% be weight or more, and a polyglycerin portion constituting the polyglycerin fatty acid monoester is composed of one or a mixture of two or more selected from di, tri, tetra and pentaglycerin and the total amount thereof is 90% by weight or more, and food utilising this preserving method.

SUMMARY

The present invention provides antimicrobial compositions and a kit having effective antimicrobial activity for reducing levels of microorganisms on both organic matter such as food and mammalian skin, and inanimate materials. Such compositions are typically useful when applied to a wide variety of surfaces. They can provide effective reduction, prevention, or elimination of microbes, particularly bacteria, fungi, and viruses. Preferably, the microbes are of a relatively wide variety such that the compositions of the present invention have a broad spectrum of activity.

Compositions of the present invention include an antimicrobial composition component, comprising propylene glycol (C7-C14) fatty acid ester in a concentration higher than any other individual component and an enhancer, wherein the enhancer is an alpha-hydroxy acid, a beta-hydroxy acid, a carboxylic acid, a chelating agent, a phenolic compound, a (C1-C10) monohydroxy alcohol, bacteriocins, antimicrobial enzymes, iron-binding proteins and derivatives thereof,

siderophones, sugars or sugar alcohols wherein the propylene glycol (C7-C14) fatty acid ester comprises monoester in an amount greater than 60%.

The invention includes antimicrobial formulations safe for use in food containing a major amount of C8-C14 propylene glycol fatty acid esters that contain at least 60% of the fatty acid monoester, an enhancer, and optionally one or more surfactants. In another aspect, the compositions may optionally also contain a surfactant. The surfactants can be chosen based on the anticipated use of the composition.

Suitable surfactants include acyl lactylate salts, dioctyl sulfosuccinate salts, lauryl sulfate salts, dodecylbenzene sulfonate salts, salts of C8-C18 fatty acids, glycerol esters, sorbitan esters, and block copolymers of polyalkylene oxide.

In a further aspect of the present invention, certain embodiments containing food-grade components exhibits effective antimicrobial activity without detrimentally affecting the taste, texture, color, odor or appearance of food and food products. This may be evaluated by using a blind taste test. For food that is normally cooked, such as hamburger, blind taste testing should be conducted on the cooked food. The treated food is considered to have no effect on taste, texture, color, odor, or appearance of food and food products, if there is no statistical difference between the treated product and a control untreated product.

In another aspect, compositions containing components that are generally recognized as food grade (GRAS), such as many of the esters and enhancers of the present invention, preferably do not pose significant harmful toxicology or environmental problems. Many of the compositions can also be readily handled at a processing plant and are compatible with processing equipment.

In another aspect of the invention, preferably at least a one-log average reduction of total aerobic bacteria count (i.e., many of which can cause food to spoil) can be achieved on substrates (e.g., food products) using the formulations and methods disclosed herein. This can be determined according to the method described in Example 6 using a sample of ground beef having an initial native bacteria concentration of 10000 -100,000 bacteria/gram ground beef when sufficient composition is applied such that 1 % antimicrobial lipid (based on meat weight %) is applied to ground beef.

More preferably the compositions of this invention achieve at least 2 log average reduction, and even more preferably at least 3 log average reduction. Most preferably, compositions of the present invention achieve complete eradication of the native bacteria (such that the bacterial level is non-detectable).

A process of disinfecting foods or other surfaces includes the step of contacting the food or surface with the concentrated composition. The compositions of the present invention can also be used for providing residual antimicrobial efficacy on a surface that results from leaving a residue or imparting a condition to the surface that remains effective and provides significant antimicrobial activity.

A method can comprise the step of diluting the composition before application to a substrate. A method can comprise the steps of applying a composition comprising an antimicrobial lipid, and separately applying an enhancer.

In another aspect of the invention, at least a one-log reduction of pathogenic bacteria can be achieved on food products using the formulations disclosed herein. In particular formulations, the compositions are not inactivated by organic matter. That is, compositions of the present invention are active in the presence of blood, serum, fats, and other organic matter typically found on food, and known to inactivate other antimicrobials such as iodine and quats..

In yet another aspect, the invention features an antimicrobial kit comprising:

> a first container with propylene glycol (C7-C14) fatty acid ester in a concentration higher than any other individual component comprising propylene glycol (C7-C14) fatty acid monoester in an amount grater than 60%;
> a surfactant; and
> a second container comprising an enhancer;
> wherein the enhancer is an alpha-hydroxy acid, a beta-hydroxy acid, a carboxylic acid, a chelating agent, a phenolic compound, a (C1-C10)monohydroxy alcohol, bateriocins, antimicrobial enzymes, iron-binding proteins and derivatives thereof, siderophores, sugars, or sugar alcohols.

The kit further can include a label or package insert indicating that contents of the first container and the second container are mixed to produce an antimicrobial formulation that is effective for reducing microbial contamination. The label or package insert further can indicate that the antimicrobial formulation can be diluted before application to food, food products, and inanimate surfaces.

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list. Other features and advantages of the invention will be apparent from the following detailed description, and from the claims.

DETAILED DESCRIPTION

The formulations can be used to treat a wide variety of substrates that are or may be contaminated by microorganisms. For example, the compositions can be used to treat steel, glass, aluminium, wood, paper, polymeric materials, Formica, rubber, paper, and textiles such as cotton, nylon, polypropylene nonwovens, and linen. For example, the compositions can be used on mammalian tissues (particularly, skin, mucosal tissue, chronic wounds, acute wounds, bums, and the like) and hard surfaces such as medical (e.g., surgical) devices, floor tiles, countertops, tubs, dishes, as well as on gloves (e.g., surgical gloves). They can also be delivered from swabs, cloth, sponges, foams, nonwovens, and paper products (e.g., paper towels and wipes), for example. For compositions comprising a major amount of the antimicrobial lipid that is liquid at room temperature, the antimicrobial lipid serves as both the active antimicrobial agent and a vehicle for the other components of the antimicrobial composition. Other uses for the compositions, such as medical applications, are described in co-pending patent application no. 10/659,571, filed on September 9, 2003.

In the compositions comprising a major amount of propylene glycol fatty acid esters, the propylene glycol fatty acid esters serve as both the active antimicrobial agent and a vehicle for the other components of the antimicrobial composition. The safety of the fatty acid esters make them useful candidates for treating food, and surfaces exposed to food, to reduce the number of human pathogens and spoilage in food. The C8-C12 fatty acid esters which may be used in the present composition include known glycerol monoesters of lauric, caprylic and capric acid and/or propylene glycol monoesters of lauric, caprylic or capric acid. These monoesters have been reported to be food grade, generally recognized as safe (GRAS) materials and have been reported to be effective as food preservatives and topical pharmaceutical agents. For example, Kabara, J. of Food Protection, 44:633-647 (1981) and Kabara, J. of Food Safety, 4: 13-25 (1982) report that LAURICEDIN™ (the glycerol monoester of lauric acid commonly referred to as monolaurin), a food grade phenolic and a chelating agent may be useful in designing food preservative systems. Fatty acid monoesters have been used for over 50 years as food grade emulsifying agents in foods such as pastry and bread dough, ice cream, margarine, and salad dressings.

The fatty acid monoesters are active against Gram positive bacteria, fungi, yeasts and lipid coated viruses but alone are not generally active against Gram negative bacteria. When the fatty acid monoesters are combined with the enhancers in the composition, the composition is active against Gram negative bacteria.

In particular, formulations of the invention can reduce the number of food borne human pathogens in meat. For example, they can be used as sprays and dips to treat meat carcasses such as beef, pork, poultry, fish, and lamb carcasses. They can also be used as sprays and dips to treat further processed meat such as ground beef, ground pork, ground chicken, ground turkey, hot dogs, sausages and lunch meats. Human food borne pathogens killed by the formulations disclosed include, for example, E. coli 0157:H7, Listeria monocytogenes, and Salmonella serovars.

Not only can the formulations be used to remove human pathogens from meat and meat products, they can also be used to help protect other foods, such as plants and plant parts, from human pathogens and pathogens that produce spoilage and adversely effect the quality and shelf life of fruits and vegetables. For example, the antimicrobial compositions of the present invention demonstrate effective kill rates against molds such as Penicillium italicum and Penicillium digitatum which cause spoilage of citrus fruit such as oranges and grapefruit.

Generally, the components in the composition, as a whole, provide an antimicrobial (including antiviral, antibacterial, or antifungal) activity having a spectrum of sufficient breadth to kill, or reduce the number to an acceptable level, of essentially most pathogenic or undesired bacteria, fungi, yeasts and lipid coated viruses. It should be understood that in the compositions of the present invention, the concentrations or amounts of the components, when considered separately, may not kill to an acceptable level, or may not kill as broad a spectrum of undesired microorganisms, or may not kill as fast; however, when used together such components provide an enhanced (preferably synergistic) antimicrobial activity (as compared to the same components used alone under the same conditions).

"Effective amount" means the amount of the antimicrobial lipid component and/or the enhancer component when in a composition, as a whole, provides an antimicrobial (including, for example, antiviral, antibacterial, or antifungal) activity that reduces, prevents, or eliminates one or more species of microbes such that an acceptable level of the microbe results. It should be understood that in the compositions of the present invention, the concentrations or amounts of the components, when considered separately, may not kill to an acceptable level, or may not kill as broad a spectrum of undesired microorganisms, or may not kill as fast; however, when used together such components provide an enhanced (preferably synergistic) antimicrobial activity (as compared to the same components used alone under the same conditions).

"Major amount" means a component present in a concentration higher than any other individual component.

"Enhancer" means a component that enhances the effectiveness of the antimicrobial lipid such that when either the composition without the antimicrobial lipid or the composition without the enhancer component are used separately, they do not provide the same level of antimicrobial activity as the composition as a whole. For example, an enhancer in the absence of the antimicrobial lipid may not provide any appreciable antimicrobial activity. The enhancing effect can be with respect to the level of kill, the speed of kill, and/or the spectrum of microorganisms killed, and may not be seen

for all microorganisms. In fact, an enhanced level of kill is most often seen in Gram negative bacteria such as Escherichia coli. An enhancer may be a synergist that when combined with the remainder of the composition causes the composition as a whole to display an activity greater than the sum of the activity of the composition without the enhancer component and the composition without the antimicrobial lipid.

"Microorganism" or "microbe" refers to bacteria, yeast, mold, fungi, mycoplasma, as well as viruses.

"Fatty" as used herein refers to a straight or branched chain alkyl or alkylene moiety having 6 to 14 (odd or even number) carbon atoms, unless otherwise specified.

The terms "comprises" and variations thereof do not have a limiting meaning where these terms appear in the description and claims.

As used herein, "a," "an," "the," "at least one," and "one or more" are used interchangeably.

Also herein, the recitations of numerical ranges by endpoints include all numbers subsumed within that range (e.g., 1 to 5 includes 1, 1.5, 2, 2.75, 3, 3.80, 4, 5, etc.).

Those of ordinary skill in the art will readily determine when a composition of the present invention provides enhanced or synergistic antimicrobial activity using assay and bacterial screening methods well known in the art. One readily performed assay involves exposing selected known or readily available viable bacterial strains, such as *Escherichia coli, Staphylococcus spp., Streptococcus spp., Pseudomonas spp.,* or *Salmonella spp.,* to a test composition at a predetermined bacterial burden level in a culture media at an appropriate temperature. After a sufficient contact time, an aliquot of a sample containing the exposed bacteria is collected, diluted, neutralized, and plated out on a culture medium such as agar. The plated sample of bacteria is incubated for about forty-eight hours and the number of viable bacterial colonies growing on the plate is counted. Once colonies have been counted, the reduction in the number of bacteria caused by the test composition is readily determined. Bacterial reduction is generally reported as $\log_{10}$ reduction determined by the difference between the $\log_{10}$ of the initial inoculum count and the $\log_{10}$ of the inoculum count after exposure.

Preferably, compositions of the invention demonstrate at least a one-log average reduction of total aerobic bacteria count when used on a substrate. To differentiate between enhanced activity and synergistic activity, a checkerboard assay can be performed.

"Shelf-Life" means a period of time it takes for a processed food to spoil. For example, beef can be considered to be spoiled if the bacterial count for an area of skin (one square centimeter) is equal to or greater than $10^7$ (colony forming units per square centimeter).

"Vehicle" means a carrier for the components of a composition. In antimicrobial compositions, the vehicle is typically the component present in a major amount.

Antimicrobial activity includes activity against microbes, including but not limited to, gram-negative bacteria and gram-positive bacteria, fungi, fungal spores, yeast, mycoplasma organisms, and lipid-coated viruses.

"Stable activity" means that the antimicrobial activity of the composition remains essentially constant or above a specified level. In some compositions, the propylene glycol fatty acid esters, and optionally glycerol fatty acid esters, may react with other components present, but the overall composition will maintain stable activity.

Preferred compositions of the present invention are physically stable. As defined herein "physically stable" compositions are those that do not significantly change due to substantial precipitation, crystallization, phase separation, and the like, from their original condition during storage at 23°C for at least 3 months, and preferably for at least 6 months. In most embodiments, the compositions will be physically stable with little or no phase separation above 4 deg C. Particularly preferred compositions are physically stable if a 10-milliliter (10-ml) sample of the composition when placed in a 15-ml conical-shaped graduated plastic centrifuge tube (Coming) and centrifuged at 3,000 revolutions per minute (rpm) for 10 minutes using a Labofuge B, model 2650 manufactured by Heraeus Sepatech GmbH, Osterode, West Germany has no visible phase separation in the bottom or top of the tube.

Preferred compositions of the present invention exhibit good chemical stability. This can be especially a concern with the antimicrobial fatty acid esters, which can often undergo transesterification, for example. In most compositions, the propylene glycol fatty acid esters are chemically stable and undergo little or no hydrolysis. Preferred compositions retain at least 85%, more preferably at least 90%, even more preferably at least 92%, and even more preferably at least 95%, of the antimicrobial lipid component after aging for 4 weeks at 50°C (an average of three samples). The most preferred compositions retain an average of at least 97% of the antimicrobial lipid after aging for 4 weeks at 50°C in a sealed container. The percent retention is understood to mean the amount of antimicrobial lipid component retained comparing the amount remaining in a sample aged in a sealed container that does not cause degradation to an identically prepared sample (preferably from the same batch) to the actual measured level in a sample prepared and allow to sit at room temperature for one to five days. For compositions that are meant to be in multiple parts, the part comprising the antimicrobial fatty acid ester preferably exhibits the above stability. The level of antimicrobial lipid component is preferably determined using gas chromatography as described in the test method included in Example 2.

*Antimicrobial Formulations*

Antimicrobial formulations of the invention comprise propylene glycol ($C_7$-$C_{14}$) fatty acid ester, one or more enhancers, and optionally one or more surfactants. The compositions can be used for reducing levels of microorganisms, including gram-negative and gram-positive bacteria, viruses, fungi and fungi spores on plants and plant parts, meat and other foods as well as on inanimate surfaces. As used herein, "reducing levels of microorganisms" includes inhibiting microbial growth, promoting microbial death, and removing microorganisms from the surfaces of plants or plant parts, meat and other foods as well as from inanimate surfaces.

Preferably, the compositions of the present invention are formulated as low viscosity liquid solutions. However, some of the compositions may be formulated in one of the following forms:

A hydrophobic ointment: The compositions are formulated with a hydrophobic base (e.g., thickened or gelled water insoluble oil) and optionally having a minor amount of a water-soluble phase.

An oil-in-water emulsion: The compositions may be formulations in which the antimicrobial lipid component is emulsified into an emulsion comprising a discrete phase of a hydrophobic component and a continuous aqueous phase that includes water and optionally one or more polar hydrophilic carrier(s) as well as salts, surfactants, emulsifiers, and other components. These emulsions may include water-soluble or water-swellable polymers as well as one or more emulsifier(s) that help to stabilize the emulsion. These emulsions generally have higher conductivity values, as described in U.S. Pat. Application Serial No. 09/966,511, filed on September 28, 2001.

A water-in-oil emulsion: The compositions may be formulations in which the antimicrobial lipid component is incorporated into an emulsion that includes a continuous phase of a hydrophobic component and an aqueous phase that includes water and optionally one or more polar hydrophilic carrier(s) as well as salts or other components. These emulsions may include oil-soluble or oil-swellable polymers as well as one or more emulsifier(s) that help to stabilize the emulsion.

Thickened Aqueous gels: These systems include an aqueous phase which has been thickened to achieve a viscosity of at least 500 centipoise (cps), more preferably at least 1,000 cps, even more preferably at least 10,000 cps, even more preferably at least 20,000 cps, even more preferably at least 50,000 cps, even more preferably at least 75,000 cps, even more preferably at least 100,000 cps, and even more preferably at least 250,000 cps (and even as high as 500,000 cps, 1,000,000 cps, or more). These systems can be thickened by suitable natural, modified natural, or synthetic polymers as described below. Alternatively, the thickened aqueous gels can be thickened using suitable polyethoxylated alkyl chain surfactants that effectively thicken the composition as well as other nonionic, cationic, or anionic emulsifier systems. Preferably, cationic or anionic emulsifier systems are chosen since some polyethoxylated emulsifiers can inactivate the antimicrobial lipids especially at higher concentrations. For certain embodiments, anionic emulsifier systems are used.

Hydrophilic vehicle: These are systems in which the continuous phase includes at least one water soluble hydrophilic component other than water. The formulations may optionally also contain water up to 20% by weight. Higher levels may be suitable in some compositions. Suitable hydrophilic components include one or more glycols such as glycerin, propylene glycol, butylene glycols, etc., polyethylene glycols (PEG), random or block copolymers of ethylene oxide, propylene oxide, and/or butylene oxide, polyalkoxylated surfactants having one or more hydrophobic moieties per molecule, silicone copolyols, as well as combinations thereof, and the like. One skilled in the art will recognize that the level of ethoxylation should be sufficient to render the hydrophilic component water soluble or water dispersible at 23°C. In most embodiments, the water content is less than 20%, preferably less than 10%, and more preferably less than 5% by weight of the composition.

Neat Compositions: The antimicrobial lipid compositions of the present invention also may be delivered to a substrate in a neat form or in a volatile solvent that rapidly evaporates to leave behind a neat composition. Such compositions may be solid, semi-solid or liquid. In the case where the compositions are solid, the antimicrobial lipid component, and/or the enhancer and/or the surfactant may optionally be microencapsulated to either sustain the delivery or facilitate manufacturing a powder which is easily delivered. Alternatively, the composition can be micronized into a fine powder without the addition of other components or it may optionally contain fillers and other ingredients that facilitate powder manufacture. Suitable powders include but are not limited to calcium carbonate, calcium phosphate, various sugars, starches, cellulose derivatives, gelatin, and polymers such as polyethylene glycols.

Alternatively, formulations can be considered which gel or thicken when warmed. For example, aqueous compositions based on Pluronic F127 (e.g., greater than about 17% by weight), as well as other Poloxamers of similar structure, are relatively low viscosity at 4°C but when warmed above 35°C can become very viscous.

Similarly, the viscosity and/or melt temperature can be enhanced by either incorporating a crystalline or semicrystalline emulsifier and/or hydrophobic carrier such as addition of an insoluble filler/thixotrope, or by addition of a polymeric thickener (e.g., a polyethylene wax in a petrolatum vehicle). Polymeric thickeners may be linear, branched,

or slightly crosslinked.

Antimicrobial Lipid

The antimicrobial lipid is that component of the composition that provides at least part of the antimicrobial activity. That is, the antimicrobial lipid has at least some antimicrobial activity for at least one microorganism. It is generally considered the main active component of the compositions of the present invention. The antimicrobial lipid component is a compound selected from the group consisting of a (C7-C14) saturated fatty acid ester of a propylene glycol (preferably, (C8-C 14)saturated fatty acid ester of a propylene glycol).

A fatty acid ester of a polyhydric alcohol is preferably of the formula ($R^1$-C(O) - O)n -$R^2$, wherein $R^1$ is the residue of a (C7-C14)saturated fatty acid (preferably, a (C8-C14)saturated fatty acid), $R^2$ is the residue of a propylene glycol, and n = 1.

Preferred fatty acid esters of propylene glycol are esters derived from C7, C8, C9, C10, C11, and C12 saturated fatty acids.

Propylene glycol monoesters of lauric, caprylic, capric andheptanoic acid, are active against Gram positive bacteria, fungi, yeasts and lipid coated viruses but alone are not generally active against Gram negative bacteria. Exemplary fatty acid monoesters include propylene glycol monoesters of lauric, caprylic, and capric acid, as well as lauric, caprylic, and capric acid monoesters of sucrose.

In certain preferred embodiments, and in particular those embodiments for use with food products, the fatty acid monoesters that are suitable for use in the present composition include known monoesters of lauric, caprylic, and capric acid, such as propylene glycol monolaurate, propylene glycol monocaprate, propylene glycol monocaprylate and combination thereof.

Propylene glycol fatty acid esters are used, these esters in the composition serve a dual purpose as both the antimicrobial active and the vehicle without the need of another aqueous or non-aqueous solvent as a separate vehicle. In preferred embodiments, when antimicrobial lipids that are solid at room temperature are used, the antimicrobial composition should be liquid at or above 4°C. In other less preferred embodiments, the composition may be a solid regardless whether the antimicrobial lipid is liquid or solid. The compositions of the present invention disclose highly concentrated antimicrobial solutions as both vehicle and active antimicrobial agent in order to deliver higher concentrations of the antimicrobial lipid to the food or other treated surface. These compositions both increase efficacy and at the same time give stable compositions and reduce costs of use.

Enhancer

Compositions of the present invention include an enhancer (preferably a synergist) to enhance the antimicrobial activity especially against Gram negative bacteria, such as E. coli. The enhancer may be an alpha-hydroxy acid, a beta-hydroxy acid, other carboxylic acids, a chelating agent other than a carboxylic acid, a phenolic compound (such as certain antioxidants and parabens), or a (C1-C10) monohydroxy alcohol. Other suitable enhancers include bacteriocins, antimicrobial enzymes, iron-binding proteins and derivatives thereof, siderophores, sugars, sugar alcohols.

The alpha-hydroxy acid, beta-hydroxy acid, and other carboxylic acid enhancers are preferably present in their protonated, free acid form. It is not necessary for all of the acidic enhancers to be present in the free acid form, however, the preferred concentrations listed below refer to the amount present in the free acid form. Furthermore, the chelator enhancers that include carboxylic acid groups are preferably present with at least one, and more preferably at least two, carboxylic acid groups in their free acid form. The concentrations given below assume this to be the case.

One enhancer may be used in the compositions of the present invention at a suitable level to produce the desired result. In a preferred embodiment, they are present in a total amount of at least 0.01 wt-%, based on the total weight of the ready to use composition. In a preferred embodiment, they are present in a total amount of no greater than 20 wt-%, based on the total weight of the ready to use composition. Such concentrations typically apply to alpha-hydroxy acids, beta-hydroxy acids, other carboxylic acids, chelating agents, phenolics, (C5-C10) monohydroxy alcohols. Generally, higher concentrations are needed for (C1-C4) monohydroxy alcohols, as described in greater detail below.

Alpha-hydroxy Acids. An alpha-hydroxy acid is typically a compound represented by the formula:

$$R^5(CR^6OH)_nCOOH$$

wherein: $R^5$ and $R^6$ are each independently H or a (C1-C8) alkyl group (straight, branched, or cyclic), a (C6-C12) aryl, or a (C6-C12) aralkyl or alkaryl group (wherein the alkyl group is straight, branched, or cyclic), wherein $R^5$ and $R^6$ may be optionally substituted with one or more carboxylic acid groups; and n = 1-3, preferably, n = 1-2.

Exemplary alpha-hydroxy acids include, but are not limited to, lactic acid, malic acid, citric acid, 2-hydroxybutanoic acid, 3-hydroxybutanoic acid, mandelic acid, gluconic acid, glycolic acid, tartaric acid, alpha-hydroxyethanoic

acid, ascorbic acid, alpha-hydroxyoctanoic acid, hydroxycaprylic acid, as well as derivatives thereof (e.g., compounds substituted with hydroxyls, phenyl groups, hydroxyphenyl groups, alkyl groups, halogens, as well as combinations thereof)). Preferred alpha-hydroxy acids include lactic acid, malic acid, and mandelic acid. These acids may be in D, L, or DL form and may be present as free acid, lactone, or partial salts thereof. Preferrably the acids are present in the free acid form. In certain preferred embodiments, the alpha-hydroxy acids useful in the compositions of the present invention are selected from the group consisting of lactic acid, mandelic acid, and malic acid, and mixtures thereof. Other suitable alpha-hydroxy acids are described in U.S. Pat. No. 5,665,776 (Yu).

One or more alpha-hydroxy acids may be used in the compositions of the present invention at a suitable level to produce the desired result. In a preferred embodiment, they are present in a total amount of at least 0.25 wt-%, more preferably, at least 0.5 wt-%, and even more preferably, at least 1 wt-%, based on the total weight of the ready to use composition. In a preferred embodiment, they are present in a total amount of no greater than 10 wt-%, more preferably, no greater than 5 wt-%, and even more preferably, no greater than 3 wt-%, based on the total weight of the ready to use composition.

Beta-hydroxy Acids. A beta-hydroxy acid is typically a compound represented by the formula:

$R^7(CR^8OH)_n(CHR^9{}_2)_mCOOH$ or

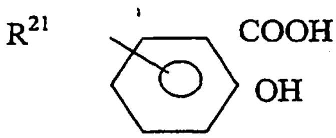

wherein: $R^7$, $R^8$, and $R^9$ are each independently H or a (C1-C8)alkyl group (saturated straight, branched, or cyclic group), a (C6-C12)aryl, or a (C6-C12)aralkyl or alkaryl group (wherein the alkyl group is straight, branched, or cyclic), wherein $R^7$ and $R^8$ may be optionally substituted with one or more carboxylic acid groups; m = 0 or 1; n = 1-3 (preferably, n =1-2); and $R^{21}$ is H, (C1-C4)alkyl or a halogen.

Exemplary beta-hydroxy acids include, but are not limited to, salicylic acid, beta-hydroxybutanoic acid, tropic acid, and trethocanic acid. In certain preferred embodiments, the beta-hydroxy acids useful in the compositions of the present invention are selected from the group consisting of salicylic acid, beta-hydroxybutanoic acid, and mixtures thereof. Other suitable beta-hydroxy acids are described in U.S. Pat. No. 5,665,776 (Yu).

One or more beta-hydroxy acids may be used in the compositions of the present invention at a suitable level to produce the desired result. In a preferred embodiment, they are present in a total amount of at least 0.1 wt-%, more preferably at least 0.25 wt-%, and even more preferably at least 0.5 wt-%, based on the total weight of the ready to use composition. In a preferred embodiment, they are present in a total amount of no greater than 10 wt-%, more preferably no greater than 5 wt-%, and even more preferably no greater than 3 wt-%, based on the total weight of the ready to use composition.

In systems with low concentrations of water, or that are essentially free of water, transesterification may be the principle route of loss of the Fatty Acid MonoEster (FAME), Fatty AlkylMonoETHer (FAMEth), and alkoxylated derivatives of these active ingredients. Thus, certain beta-hydroxy acids (BHA) are particularly preferred since these are believed to be less likely to transesterify the ester antimicrobial lipid or other ester by reaction of the hydroxyl group of the AHA or BHA. For example, salicylic acid may be particularly preferred in certain formulations since the phenolic hydroxyl group is a much more acidic alcohol and thus much less likely to react.

Other Carboxylic Acids. Carboxylic acids other than alpha- and beta-carboxylic acids are suitable for use as an enhancer. These include alkyl, aryl, aralkyl, or alkaryl carboxylic acids typically having equal to or less than 18 carbon atoms. A preferred class of these can be represented by the following formula:

$$R^{10}(CR^{11})_nCOOH$$

wherein: $R^{10}$ and $R^{11}$ are each independently H or a (C1-C4) saturated or unsaturated aliphatic group (which can be a straight, branched, or cyclic group), a (C6-C12)aryl group, a (C6-C 18) group containing both aryl groups and aliphatic groups (which can be a straight, branched, or cyclic group), wherein $R^{10}$ and $R^{11}$ may be optionally substituted with one or more carboxylic acid groups; and n = 0-3, preferably, n = 0-2.

Exemplary acids include, but are not limited to, acetic acid, propionic acid, benzoic acid, benzylic acid, nonylbenzoic acid, and the like. Particularly preferred is benzoic acid.

One or more carboxylic acids may be used in the compositions of the present invention at a suitable level to produce the desired result. In a preferred embodiment, they are present in a total amount of at least 0.1 wt-%, more

preferably at least 0.25 wt-%, even more preferably at least 0.5 wt-%, and most preferably at least 1 wt-%, based on the ready to use concentration composition. In a preferred embodiment, they are present in a total amount of no greater than 10 wt-%, more preferably no greater than 5 wt-%, and even more preferably no greater than 3 wt-%, based on the ready to use composition.

Chelators. A chelating agent (i.e., chelator) is typically an organic compound capable of multiple coordination sites with a metal ion in solution. Typically these chelating agents are polyanionic compounds and coordinate best with polyvalent metal ions. Exemplary chelating agents include, but are not limited to, ethylene diamine tetraacetic acid (EDTA) and salts thereof (e.g., EDTA$(Na)_2$, EDTA$(Na)_4$, EDTA(Ca), EDTA$(K)_2$), sodium acid pyrophosphate, acidic sodium hexametaphosphate, adipic acid, succinic acid, polyphosphoric acid, sodium acid pyrophosphate, sodium hexametaphosphate, acidified sodium hexametaphosphate, nitrilotris(methylenephosphonic acid), diethylenetriaminepentaacetic acid, 1-hydroxyethylene, 1,1-diphosphonic acid, and diethylenetriaminepenta-(methylenephosphonic acid). Certain carboxylic acids, particularly the alpha-hydroxy acids and beta-hydroxy acids, can also function as chelators, e.g., malic acid and tartaric acid.

Also included as chelators are compounds highly specific for binding ferrous and/or ferric ion such as siderophores, and iron binding proteins. Iron binding protein include, for example, lactoferrin, and transferrin. Siderophores include, for example, enterochlin, enterobactin, vibriobactin, anguibactin, pyochelin, pyoverdin, and aerobactin.

In certain preferred embodiments, the chelating agents useful in the compositions of the present invention include those selected from the group consisting of ethylenediaminetetraacetic acid and salts thereof, succinic acid, and mixtures thereof. Preferably, either the free acid or the mono- or di-salt form of EDTA is used.

One or more chelating agents may be used in the compositions of the present invention at a suitable level to produce the desired result. In a preferred embodiment, they are present in a total amount of at least 0.01 wt-%, more preferably at least 0.05 wt-%, even more preferably at least 0.1 wt-%, and even more preferably at least 1 wt-%, based on the weight of the ready to use composition. In a preferred embodiment, they are present in a total amount of no greater than 10 wt-%, more preferably no greater than 5 wt-%, and even more preferably no greater than 1 wt-%, based on the weight of the ready to use composition.

Phenolic Compounds. A phenolic compound enhancer is typically a compound having the following general structure:

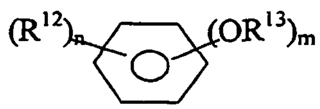

wherein: m is 0 to 3 (especially 1 to 3), n is 1 to 3 (especially 1 to 2), each $R^{12}$ independently is alkyl or alkenyl of up to 12 carbon atoms (especially up to 8 carbon atoms) optionally substituted with O in or on the chain (e.g., as a carbonyl group) or OH on the chain, and each $R^{13}$ independently is H and alkyl or alkenyl of up to 8 carbon atoms (especially up to 6 carbon atoms) optionally substituted with O in or on the chain (e.g., as a carbonyl group) or OH on the chain, but where $R^{13}$ is H, n preferably is 1 or 2.

Examples of phenolic enhancers include, but are not limited to, butylated hydroxy anisole, e.g., 3(2)-tert-butyl-4-methoxyphenol (BHA), 2,6-di-tert-butyl-4-methylphenol (BHT), 3,5-di-tert-butyl-4-hydroxybenzylphenol, 2,6-di-tert-4-hexylphenol, 2,6-di-tert-4-octylphenol, 2,6-di-tert-4-decylphenol, 2,6-di-tert-butyl-4-ethylphenol, 2,6-di-tert-4-butylphenol, 2,5-di-tert-butylphenol, 3,5-di-tert-butylphenol, 4,6-di-tert-butyl-resorcinol, methyl paraben (4-hydroxybenzoic acid methyl ester), ethyl paraben, propyl paraben, butyl paraben, 2-phenoxyethanol, as well as combinations thereof. A preferred group of the phenolic compounds is the phenol species having the general structure shown above where $R^{13}$ = H and where $R^{12}$ is alkyl or alkenyl of up to 8 carbon atoms, and n is 0, 1, 2, or 3, especially where at least one $R^{12}$ is butyl and particularly tert-butyl, and especially the non-toxic members thereof. Some of the preferred phenolic synergists are BHA, BHT, methyl paraben, ethyl paraben, propyl paraben, and butyl paraben as well as combinations of these.

One or more phenolic compounds may be used in the compositions of the present invention at a suitable level to produce the desired result. The concentrations of the phenolic compounds in medical-grade compositions may vary widely, but as little as 0.001 wt-%, based on the total weight of the composition, can be effective when the above-described esters are present within the above-noted ranges. In a preferred embodiment, they are present in a total amount of at least 0.01 wt-%, more preferably at least 0.10 wt-%, and even more preferably at least 0.25 wt-%, based on the ready to use composition. In a preferred embodiment, they are present in a total amount of no greater than 8 wt-%, more preferably no greater than 4 wt-%, and even more preferably no greater than 2 wt-%, based on the ready to use composition.

The above-noted concentrations of the phenolics are normally observed unless concentrated formulations for subsequent dilution are intended. On the other hand, the minimum concentration of the phenolics and the antimicrobial lipid components to provide an antimicrobial effect will vary with the particular application.

Monohydroxy Alcohols. An additional enhancer is a monohydroxy alcohol having 1-10 carbon atoms. This includes the lower (i.e., C1-C4) monohydroxy alcohols (e.g., methanol, ethanol, isopropanol, and butanol) as well as longer chain (i.e., C5-C10) monohydroxy alcohols (e.g., iosbutanol, t-butanol, octanol, and decanol). In certain preferred embodiments, the alcohols useful in the compositions of the present invention are selected from the group consisting of methanol, ethanol, isopropyl alcohol, and mixtures thereof

One or more alcohols may be used in the compositions of the present invention at a suitable level to produce the desired result. In a preferred embodiment, the short chain (i.e., C1-C4) alcohols are present in a total amount of at least 15 wt-%, more preferably at least 20 wt-%, and even more preferably, at least 25 wt-%, based on the ready to use composition. In another preferred embodiment longer chain (i.e., C5-C10) alcohols are present in a total amount of at least 0.1 wt-%, more preferably at least 0.25 wt-%, and even more preferably at least 0.5 wt-%, and most preferably at least 1.0%, based on the ready to use composition. In a preferred embodiment, the (C5-C10) alcohols are present in a total amount of no greater than 10 wt%, more preferably no greater than 5 wt-%, and even more preferably no greater than 2 wt-%, based on the total weight of the ready to use composition.

When the enhancer is an alcohol such as isopropanol or ethanol, the minimum concentration that maintains synergistic antimicrobial activity is about 15 wt% (e.g. 20-30 wt% for ethanol and 15-20 wt% for isopropanol). For longer chain alcohols such as decyl alcohol, the minimum concentration that maintains synergistic activity is about 1 wt% (e.g. 1-2 wt%), while for 1-octanol, the minimal concentration is about 0.5 wt% (e.g. 0.5-1.0 wt%).

Surfactants

Compositions of the present invention can include a surfactant to emulsify the composition and to help wet the surface to aid in contacting the microorganisms. As used herein the term "surfactant" means an amphiphile which is defined as a molecule possessing both polar and nonpolar regions which are convalently bound. The term is meant to include soaps, detergents, emulsifiers, surface active agents and the like. The surfactant can be cationic, anionic, nonionic, or zwitterionic. This includes a wide variety of conventional surfactants; however, certain ethoxylated surfactants may reduce or eliminate the antimicrobial efficacy of the antimicrobial lipid. The exact mechanism of this is not known and not all ethoxylated surfactants display this negative effect. For example, poloxamer polyethylene oxide/polypropylene oxide surfactants have been shown to be compatible with the antimicrobial lipid component, but ethoxylated sorbitan fatty acid esters such as those sold under the trade name TWEEN by ICI have not been compatible in some formulations. It should be noted that these are broad generalizations and the activity can be formulation dependent, i.e., based on the selection and amount of both antimicrobial lipid and ethoxylated surfactant used. One skilled in the art can easily determine compatibility of a surfactant by making the formulation and testing for antimicrobial activity as described in the Examples Section. Combinations of various surfactants can be used if desired.

Examples of the various classes of surfactants are described below. In certain preferred embodiments, the surfactants useful in the compositions of the present invention are selected from the group consisting of sulfonates, sulfates, phosphonates, phosphates, poloxamer (polyethylene oxide/polypropylene oxide block copolymers), cationic surfactants, and mixtures thereof. In certain more preferred embodiments, the surfactants useful in the compositions of the present invention are selected from the group consisting of sulfonates, sulfates, phosphates, and mixtures thereof.

One or more surfactants may be used in the compositions of the present invention at a suitable level to produce the desired result. In a preferred embodiment, they are present in a total amount of at least 0.1 wt%, more preferably, at least 0.5 wt-%, and even more preferably, at least 1.0 wt-%, based on the total weight of the ready to use composition. In a preferred embodiment, they are present in a total amount of no greater than 10 wt-%, more preferably, no greater than 5 wt-%, and even more preferably, no greater than 2 wt-%, based on the total weight of the ready to use composition. The ratio of the total concentration of surfactant to the total concentration of the antimicrobial lipid component is preferably within a range of 5:1 to 1:100, more preferably 3:1 to 1:10, and most preferably 2:1 to 1:3, on a weight basis.

Cationic Surfactants. Exemplary cationic surfactants include, but are not limited to, salts of primary, secondary or tertiary fatty amines and their polyoxyalkylenated derivatives thereof; quaternary ammonium salts such as tetraalkylammonium, alkylamidoalkyltrialkylammonium, trialkylbenzylammonium, trialkylhydroxyalkylammonium or alkylpyridinium halides (preferably chlorides or bromides); imidazoline derivatives; amine oxides of a cationic nature (e.g., at an acidic pH).

In certain preferred embodiments, the cationic surfactants useful in the compositions of the present invention are selected from the group consisting of tetralkyl ammonium, trialkylbenzylammonium, and alkylpyridinium halides, and mixtures thereof.

Also particularly preferred are amine oxide surfactants including alkyl and alkylamidoalkyldialkylamine oxides of the following formula:

$$(R^{14})_3\text{-N}\rightarrow\text{O}$$

wherein $R^{14}$ is a (C1-C22)alkyl group (preferably a (C1-C14)alkyl group) or a (C6-C18)aralldyl or (C6-C18)alkaryl group, wherein any of these groups can be optionally substituted in or on the chain by N, O, S including groups such as amide, ester, hydroxyl, and the like. Each $R^{14}$ may be the same or different provided at least one $R^{14}$ group includes at least eight carbons. Optionally, the $R^{14}$ groups can be joined to form a heterocyclic ring with the nitrogen to form surfactants such as amine oxides of alkyl morpholine, alkyl piperazine, and the like. Preferably two $R^{14}$ groups are methyl and one $R^{14}$ group is a (C12-C16)alkyl or alkylamidopropyl group.

Anionic Surfactants. Exemplary anionic surfactants include, but are not limited to, sarcosinates, glutamates, alkyl sulfates, araalkyl sulfates, sodium alkyleth sulfates, ammonium alkyleth sulfates, ammonium laureth-n-sulfates, laureth-n-sulfates, isethionates, glycerylether sulfonates, sulfosuccinates, alkylglyceryl ether sulfonates, alkyl phosphates, aralkyl phosphates, alkylphosphonates, and aralkylphosphonates. These anionic surfactants may have a metal or organic ammonium counterion. In certain preferred embodiments, the anionic surfactants useful in the compositions of the present invention are selected from the group consisting of:

*1. Sulfonates and Sulfates.* Suitable anionic surfactants include sulfonates and sulfates such as alkyl sulfates, alkylether sulfates, alkyl sulfonates, alkylether sulfonates, alkylbenzene sufonates, alkylbenzene ether sulfates, alkylsulfoacetates, secondary alkane sulfonates, secondary alkylsulfates and the like. Many of these can be represented by the formulas:

$$R^{14}\text{-}(OCH_2CH_2)_n(OCH(CH_3)CH_2)_p\text{-}(Ph)_a\text{-}(OCH_2CH_2)_m\text{-}(O)_b\text{-}SO_3^{-}M^{+}$$

and

$$R^{14}\text{-}CH[SO_3\text{-}M^{+}]\text{-}R^{15}$$

wherein: a and b = 0 or 1; n, p, m = 0-100 (preferably 0-20, and more preferably 0-10); $R^{14}$ is defined as above provided at least one $R^{14}$ or $R^{15}$ is at least C8; $R^{15}$ is a (C1-C12)alkyl group (saturated straight, branched, or cyclic group) that may be optionally substituted by N, O, or S atoms or hydroxyl, carboxyl, amide, or amine groups; Ph = phenyl; and M is a cationic counterion such as H, Na, K, Li, ammonium, a protonated tertiary amine such as triethanolamine or a quaternary ammonium group.

In the formula above, the ethylene oxide groups (i.e., the "n" and "m" groups) and propylene oxide groups (i.e., the "p" groups) can occur in reverse order as well as in a random, sequential, or block arrangement. Preferably for this class, $R^{14}$ includes an alkylamide group such as $R^{16}\text{-}C(O)N(CH_3)CH_2CH_2\text{-}$ as well as ester groups such as - OC (O)-$CH_2$- wherein $R^{16}$ is a (C8-C22)alkyl group (branched, straight, or cyclic group).

*2. Phosphates and Phosponates.* Suitable anionic surfactants also include phosphates such as alkyl phosphates, alkylether phosphates, aralkylphosphates, and aralkylether phosphates. Many may be represented by the formula:

$$[R^{14}\text{-}(Ph)_a\text{-}O(CH_2CH_2O)_n(CH_2CH(CH_3)O)_p]_q\text{-}P(O)[O^{-}M^{+}]_r$$

wherein: Ph, $R^{14}$, a, n, p, and M are defined above; r is 0-2; and q =1-3; with the proviso that when q =1, r = 2, and when q = 2, r = 1, and when q = 3, r = 0. As above, the ethylene oxide groups (i.e., the "n" groups) and propylene oxide groups (i.e., the "p" groups) can occur in reverse order as well as in a random, sequential, or block arrangement.

Amphoteric Surfactants. Surfactants of the amphoteric type include surfactants having tertiary amine groups, which may be protonated, as well as quaternary amine containing zwitterionic surfactants. Those that have been particularly useful include:

*1. Ammonium Carboxylate Amphoterics.* This class of surfactants can be represented by the following formula:

$$R^{17}\text{-}(C(O)\text{-}NH)_a\text{-}R^{18}\text{-}N^{+}(R^{19})_2\text{-}R^{20}\text{-}COO^{-}$$

wherein: a = 0 or 1; $R^{17}$ is a (C7-C21)alkyl group (saturated straight, branched, or cyclic group), a (C6-C22)aryl group, or a (C6-C22)aralkyl or alkaryl group (saturated straight, branched, or cyclic alkyl group), wherein $R^{17}$ may be optionally substituted with one or more N, O, or S atoms, or one or more hydroxyl, carboxyl, amide, or amine groups; $R^{19}$ is H or a (C1-C8)alkyl group (saturated straight, branched, or cyclic group), wherein $R^{19}$ may be optionally substituted with one or more N, O, or S atoms, or one or more hydroxyl, carboxyl, amine groups, a (C6-C9)aryl group, or a (C6-C9)aralkyl or alkaryl group; and $R^{18}$ and $R^{20}$ are each independently a (C1-C10)alkylene group that may be the same or different and may be optionally substituted with one or more N, O, or S atoms, or one or more hydroxyl or amine groups.

More preferably, in the formula above, $R^{17}$ is a (C1-C18)alkyl group, $R^{19}$ is a (C1-C2)alkyl group preferably substituted with a methyl or benzyl group and most preferably with a methyl group. When $R^{19}$ is H it is understood that the surfactant at higher pH values could exist as a tertiary amine with a cationic counterion such as Na, K, Li, or a quaternary amine group.

*2. Ammonium Sulfonate Amphoterics.* This class of amphoteric surfactants are often referred to as "sultaines" or "sulfobetaines" and can be represented by the following formula

$$R^{17}\text{-(C(O)-NH)}_a\text{-}R^{18}\text{-N}^+(R^{19})_2\text{-}R^{20}\text{-SO}_3^-$$

wherein $R^{17}$-$R^{20}$ and "a" are define above. The sulfoamphoterics may be preferred over the carboxylate amphoterics since the sulfonate group will remain ionized at much lower pH values.

Nonionic Surfactants. Exemplary nonionic surfactants include, but are not limited to, alkyl glucosides, alkyl polyglucosides, polyhydroxy fatty acid amides,, sucrose esters, esters of fatty acids and polyhydric alcohols, fatty acid alkanolamides, ethoxylated fatty acids, ethoxylated aliphatic acids, ethoxylated fatty alcohols (e.g., octyl phenoxy polyethoxyethanol available under the trade name TRITON X-100 and nonyl phenoxy poly(ethyleneoxy) ethanol available under the trade name NONIDET P-40, both from Sigma, St. Louis, MO), ethoxylated and/or propoxylated aliphatic alcohols (e.g., that available under the trade name PLUROINC F127 from Sigma), ethoxylated glycerides, ethoxylated block copolymers with ethylene diaminetetraacetic acid (EDTA), ethoxylated cyclic ether adducts, ethoxylated amide and imidazoline adducts, ethoxylated amine adducts, ethoxylated mercaptan adducts, ethoxylated condensates with alkyl phenols, ethoxylated nitrogen-based hydrophobes, ethoxylated polyoxypropylenes, polymeric silicones, fluorinated surfactants (e.g., those available under the trade names FLUORAD-FS 300 from Minnesota Mining and Manufacturing Co., St. Paul, MN, and ZONYL from Dupont de Nemours Co., Wilmington, DE), and polymerizable (reactive) surfactants (e.g., SAM 211 (alkylene polyalkoxy sulfate surfactant available under the trade name MAZON from PPG Industries, Inc., Pittsburgh, PA. In certain preferred embodiments, the nonionic surfactants useful in the compositions of the present invention are selected from the group consisting of Poloxamers such as Pluronic from BASF, sorbitan fatty acid esters, and mixtures thereof. Formulations in Food Applications

The formulations of the invention are particularly useful for reducing levels of food borne human pathogens, including Escherichia coli 0157:H7, Salmonella serotypes, including S. typhimurium, Listeria (e.g., L. monocytogenes), Campylobacter (e.g., C. jejuni), Shigella species, and Bacillus cereus.

Fatty acid monoesters suitable for use in the antimicrobial formulations generally are considered food grade, GRAS, and/or are U.S. Food and Drug Administration (FDA)-cleared food additives. In particular, one or more fatty acid monoesters derived from C7 to C 12 fatty acids (preferably, C8 to C12 fatty acids) such as, propylene glycol monoesters of caprylic, capric, heptanoic or lauric acid are useful in formulations of the invention. Combinations of fatty acid monoesters can be tailored to the target microorganism. For example, laurate monoesters can be combined with caprylate monoesters and/or caprate monoesters when it is desired to reduce levels of fungi on the surface of a plant or plant part.

Propylene glycol monocaprylate (PG-C8), propylene glycol monocaprate (PG-C10), and propylene glycol monolaurate (PG-C12) are available from Uniquema International, Chicago, IL.

In food applications, the enhancers are food grade, GRAS listed, and/or FDA-cleared food additives. Suitable organic acids can include, for example, lactic acid, tartaric acid, adipic acid, succinic acid, citric acid, ascorbic acid, glycolic acid, malic acid, mandelic acid, acetic acid, sorbic acid, benzoic acid, and salicylic acid. Suitable chelating agents can include, for example, sodium acid pyrophosphate, acidic sodium hexametaphosphate (such as SPORIX acidic sodium hexametaphosphate), ethylenediaminetetraacetic acid (EDTA) and salts thereof. Suitable alcohols can be, for example, ethanol, isopropanol, or long chain alcohols such as octanol or decyl alcohol. Phenolic compounds such as butylated hydroxyanisole, butylated hydroxytoluene, and tertiary butyl hydroquinone, for example, act synergistically with the fatty acid monoesters as do benzoic acid derivatives such as methyl, ethyl, propyl, and butyl parabens.

The amounts of acid or chelating agent in the present invention which are used to provide a concentrated composition are typically up to 20.0 wt. % and preferably about 1.0-10.0 wt. %. When used, the concentrate can be diluted with a vehicle to provide an acid or chelating agent concentration of between about 0.01-1.0 wt. % and preferably about 0.01-0.5 wt. %. Lower concentrations of enhancer may be necessary, in part, in order to avoid undesired changes or alterations to the taste, texture, color, odor or appearance of the food. Depending on the particular enhancer used, it can either be formulated directly into the concentrate vehicle if soluble and stable in the esters or it can be packaged separately in a suitable solvent.

Antimicrobial formulations also can include one or more surfactants, which can facilitate dissolving or dispersing of the monoesters in water when concentrates are diluted and/or help to loosen or remove attached microorganisms from surfaces of the food and other substrates so that the microorganisms can be more readily contacted and destroyed by the formulations. Anionic surfactants, cationic surfactants, nonionic surfactants and amphoteric surfactants can be used to make suitable emulsions of the antimicrobial fatty acid esters. For example, an antimicrobial formulation can

include anionic surfactants such as acyl lactylate salts, dioctyl sulfosuccinate salts, lauryl sulfate salts, dodecylbenzene sulfonate salts, and salts of C8-C18 fatty acids. Suitable salts include sodium, potassium, or ammonium salts. Acyl lactylates include, for example, calcium or sodium stearoyl-2-lactylate, sodium isostearoyl-2-lactylate, sodium lauroyl-2-lactylate, sodium caproyl lactylate, sodium cocoyl lactylate, and sodium behenoyl lactylate. Nonionic surfactants include glycerol esters such as decaglyceryl tetraoleate; sorbitan esters such as sorbitan monolaurate, commercially available as SPAN™ 20 from Uniquema International, Chicago, IL; and block copolymers of polyalkylene oxide, e.g., polyethylene oxide and polypropylene oxide available as Pluronics™ and Tetronics™ from BASF (Parsippany, NJ). Dioctyl sodium sulfosuccinate is commercially available as GEMTEX™ SC40 surfactant (40% dioctyl sodium sulfosuccinate in isopropanol) from Finetex Inc., Spencer, North Carolina. Sodium caproyl lactylate is commercially available as PATIONIC™ 122A from RITA (Woodstock, IL). Sodium lauryl sulfate is commercially available from Stepan Chemical Co., Northfield, IL.

In most compositions, food grade and/or GRAS surfactants are used in amounts which provide a concentrated composition of between about 1.0-30.0 wt. % and preferably about 4.0-12. 0 wt. %. When used, the concentrate can be diluted in a vehicle to provide a surfactant concentration of between about 0.001-1.0 wt. % and preferably 0.01-0.5 wt. %.

The concentration of the aforementioned components required for effectively inhibiting microbial growth depends on the type of microorganism targeted and the formulation used (e.g., the type of antimicrobial lipid, enhancer and surfactants that are present). The concentrations or amounts of each of the components, when considered separately, do not kill as great a spectrum of pathogenic or undesired microorganisms, kill them as rapidly, or reduce the number of such microorganisms to an acceptable level, as the composition as a whole. Thus, the components of the formulation, when used together, provide a synergistic antimicrobial activity to the meat, plants or plant parts, or other treated surfaces when compared to the same components used alone and under the same conditions. Acceptable levels of antimicrobial activity typically exceed 1-log reduction in or on a food, or other surface.

Effective amounts of each component can be readily ascertained by one of skill in the art using the teachings herein and assays known in the art. The propylene glycol esters may be present in the concentrated antimicrobial compositions in a range from 60 to 90 wt. % of the composition

The amount of fatty acid monoesters in the propylene glycol fatty acid esters of the present invention is at least 60 wt %.

The compositions comprising a major amount of propylene glycol esters can contain fatty acid monoesters, surfactants, enhancers and other ingredients that are soluble/miscible in the propylene glycol esters. The final concentrate formulations preferably stay transparent, e.g., remain in a single phase state, for at least one day at room temperature. Some concentrations may have phase separation at room temperature but return to single phase at higher temperature, such as 40°C or 50°C.

The concentrated formulations of the invention can be prepared and used directly or can be diluted to prepare a non-aqueous or aqueous solution, emulsion or suspension before use. Suitable vehicles for preparing the solutions or suspensions are typically safe and acceptable to regulatory agencies such as the FDA and the U.S. Environmental Protection Agency (EPA). Particularly acceptable vehicles include water, propylene glycol, polyethylene glycol, glycerin, ethanol, isopropanol, and combinations thereof. Alternatively, one or more antimicrobial lipids may function as the vehicle.

When diluted before use, the fatty acid monoglyceride is about 0.001 to 30 weight % (wt%), the enhancer is about 0.001 to 30 wt%, and one or more surfactants are 0.001 to 30 wt% of the antimicrobial formulation. For example, a ready-to-use formulation can include 0.01 to 5.0 wt% of a fatty acid monoester, about 0.5 to 30 wt% of an enhancer, and about 0.5 wt% to 5.0 wt% of a surfactant. In particular, a ready-to-use formulation can include about 0.2 wt% to about 2.0 wt% of the fatty acid monoester, about 0.1 wt% to about 25.0 wt% of the enhancer, and about 0.1 wt% to about 1.5 wt% of one or more surfactants.

Additional components of the antimicrobial formulations can include, for example, food-grade coating agents such as food-grade waxes, i.e., bees wax, paraffin, carnauba, candelilla, polyethylene waxes; other coating materials such as resins, shellac, wood rosin, and corn zein; components that protect the formulations from UV inactivation or degradation, colorants, odor-enhancing agents, viscosity control agents such as gum tragacanth, gum accacia, carageenans, Carbopols (B.F. Goodrich, Cleveland, Ohio), guar gum, and cellulose gums; anti-foaming agents such as silicone anti-foams, e.g., polydimethylsiloxanes (Dow Corning, Midland, MI), sticking agents, or flavorants such as natural oils or artificial sweeteners.

The concentrated compositions of the present invention exhibit little or no phase separation of the composition between 4 and 80 deg C. Some compositions will phase separate at 4 deg C but will return to single phase when heated.

Antimicrobial formulations used in food applications typically exhibit increased antimicrobial efficacy with increased temperatures at application.

*Treating Meat and Meat Products*

The composition of the present invention may be prepared by combining the above described components using processes and procedures well known to those of ordinary skill in the art. For example, a concentrated composition

is prepared by heating a propylene glycol fatty acid ester to 70°C., adding a surfactant, and then adding an enhancer soluble in the fatty acid ester to form a solution. In some embodiments, the antimicrobial lipid can be applied in a separate step from applying the enhancer.

The compositions of the present invention may be used in a food processing plant in a variety of suitable ways during various stages of the process. For example, the present composition may be applied to meat products, such as beef carcasses, beef trim, beef primals, or ground meat as a spray, a rinse, or a wash solution. The meat products may also be dipped in the composition. In addition, the present invention has a wide useful temperature range which allows the composition to be used at different stages in a process plant. For example, the composition may be used at elevated temperatures to disinfect beef carcasses and at cold (4-5 deg C) temperatures to disinfect ground beef and beef trim. Also, if the meat or meat product is cooked, compositions of the present invention can be particularly effective. The compositions of the present invention may also be useful in the products and processes disclosed in U.S. Patent Nos. 5,460,833 and 5,490,992.

*Treating Plants and Plant Parts*

Using the formulations of the present invention, levels of plant pathogens can be reduced on the surfaces of plants and plant parts, which can extend shelf life of the plants and plant parts. Non-limiting examples of plant pathogens include *Erwinia carotovora, Fusarium* species, *Botrytis* species, *Phytopthera* species, *Phoma* species, *Verticilium* species, *Penicillium* species, and *Colletotrichum* species. The formulations of the invention also are effective at reducing viability of spores on surfaces of plants and plant parts, such as spores from penicillium fungi.

Formulations of the invention can be applied to plants and plant parts by, for example, spraying, dipping, wiping, brushing, sponging, or padding. The formulation can be applied to a portion of or over the entire exterior surface of a plant or plant part. In most applications, the entire surface of the plant or plant part is fully wetted with the formulation. In some embodiments, the antimicrobial lipid can be applied in a separate step from applying the enhancer.

Formulations can be applied at temperatures ranging from 2°C to 90°C and are in contact with the surface of the plant or plant part for a time sufficient to reduce microbial levels (e.g. 10 seconds to 60 minutes). Typically, application time is reduced as temperature is increased. Heating the formulation to between 40°C and 65°C (e.g., 44-60°C, 46-58°C, 48-56°C, or 50-54°C) and applying to the surface while still warm is particularly effective for reducing microbial levels on plants or plant parts. If present, the liquid vehicle can be removed from the surface of plant or plant part by, for example, air drying. Also, if the plant or plant part is cooked, compositions of the present invention can be particularly effective.

Suitable plants and plant parts include raw agricultural commodities (i.e., non-processed products) and processed products. Non-limiting examples of raw agricultural commodities include alfalfa seeds, sprouts, cucumbers, melons, onions, lettuce, cabbage, carrots, potatoes, eggplants, citrus fruits such as grapefruits, lemons, limes, and oranges, bananas, pineapples, kiwis, and apples. Processed products include torn, sliced, chopped, shredded, or minced fruits or vegetables, as well as juice obtained from fruits or vegetables.

For example, a fruit such as an orange can be treated with an antimicrobial formulation of the invention, air-dried, then coated with a food-grade wax. This produces an orange having the antimicrobial formulation interposed between the orange and the food-grade coating. Alternatively, the antimicrobial formulation and a food-grade coating can be intermixed prior to application. In another alternative, the food-grade wax may be applied to fruit, such as an orange, and then the fruit can be treated with the antimicrobial composition over the wax.

The compositions of the present invention may also be useful in the products and processes disclosed in publication WO 200143549A.

Formulations and Methods of Preparation

It will also be appreciated that additional antiseptic, disinfectants, or antibiotics may be included and are contemplated. These include, for example, oxidizing agents such as ozone; chlorine compounds such as sodium hypochlorite, chloride dioxide); salts such as trisodium phosphate and acidic calcium sulfate; addition of metals such as silver, copper, zinc; iodine and iodophors; chlorhexidine and its various salts such as chlorhexidine digluconate; polyhexamethylenebiguanide, parachlorometaxylenol, triclosan, antimicrobial quaternary amines including polymeric quaternary amines, "azole" antifungal agents including clortrimazole, miconazole, econazole, ketoconazole, and salts thereof; and the like. Antibiotics such as neomycin sulfate, bacitracin, mupirocin, polymyxin, rifampin, tetracycline, and the like, also may be included.

Examples of other suitable antiseptics include, for example, peroxides, (C6-C14)alkyl carboxylic acids and alkyl ester carboxylic acids, antimicrobial natural oils, as described in Applicants' Assignee's Copending U.S.-A-2006/0051384 , filed on September 7, 2004; halogenated phenols, diphenyl ethers, bisphenols (including but not limited to p-chloro m-xylenol (PCMX) and triclosan), and halogenated carbanilides described in Applicants' Assignee's Copending U.S. -A-2006/0052452 filed on September 7, 2004; digluconate, diacetate, dimethosulfate, and dilactate salts; polymeric quaternary ammonium compounds such as polyhexamethylenebiguanide; silver and various silver complexes; small molecule quaternary ammonium compounds such as benzalkoium chloride and alkyl substituted derivatives; quaternary ammonium compounds with at least one alkyl (C8-C18)chain; cetylpyridinium halides and their derivatives; benzathonium chloride and its alkyl substituted derivatives; and octenidine described in Applicants' Assignee's Copending U.S.-A-2006/0051385, filed on September 7, 2004; and compatible combinations thereof.

It may also be suitable to include preservatives in the formulation to prevent growth of certain organisms. Suitable preservatives include industry standard compounds such as parabens (methyl, ethyl, propyl, isopropyl, isobutyl, etc), 2 bromo-2 nitro-1,3 diol; 5 bromo-5-nitro-1,3 dioxane, chlorbutanol, diazolidinyl urea; iodopropylnyl butylcarbamate, phenoxyethanol, halogenated cresols, methylchloroisothiazolinone and the like, as well as combinations of these compounds.

The formulations are typically selected from one of the following five types: (1) formulations with a hydrophobic vehicle which may be anhydrous, nearly anhydrous or further comprise a aqueous phase; (2) formulations based on water in oil emulsions in which the water insoluble continuous "oil" phase is comprised of one or more hydrophobic components; (3) formulations with a hydrophilic vehicle which may be anhydrous, nearly anhydrous or further comprise a aqueous phase; (4) highly viscous water-based formulations which may be solutions or oil in water emulsions; and (5) neat compositions which are essentially free of a hydrophobic or hydrophilic vehicle component comprising antimicrobial limpid optionally an enhancer, and further optionally a surfactant In this latter case the compositions may optionally be dissolved in a volatile carrier solvent for delivery to the intended substrate or maybe delivered to the site as a dry powder, liquid, or semi-solid composition. The different types of compositions are discussed further below.

(1) Anhydrous or Nearly Anhydrous Formulations with a Hydrophobic Vehicle: In certain preferred embodiments of the present invention, the compositions include an antimicrobial lipid component in a hydrophobic vehicle optionally in combination with surfactant(s), an enhancer component, and a small amount of a hydrophilic component. In most instances the enhancers are not soluble in the hydrophobic component at room temperature although they may be at elevated temperatures. The hydrophilic component is generally present in a sufficient amount to stabilize (and perhaps to solubilize) the enhancer(s) in the composition. It is believed that these formulations produce an emulsion in which the enhancer and/or surfactant is dissolved, emulsified, or dispersed in the hydrophilic component which is emulsified into the hydrophobic component(s). These compositions are stable upon cooling and centrifuging.

The water content of these formulations is preferably less than 20 wt-%, more preferably less than 10 wt-%, and even more preferably less than 5 wt-%, and most preferably less than 2 wt-%, in order to minimize chemical degradation of antimicrobial lipids present as well as to reduce concerns with microbial contamination in the composition during storage.

(2) Water in Oil Emulsions: Antimicrobial lipid components of this invention can be formulated into water-in-oil emulsions in combination with enhancer(s) and surfactant(s). Particularly preferred compositions comprise at least 35%, preferably at least 40%, more preferably at least 45% and most preferably at least 50% by weight oil phase. As used herein the oil phase is comprised of all components which are either not soluble in water or preferentially soluble in the oil(s) present at 23°C.

(3) Hydrophilic Vehicle: Antimicrobial lipid components of this invention can be formulated into a hydrophilic component such as that based on the hydrophilic compounds discussed above optionally in combination with the enhancer(s) and surfactant(s). Particularly preferred are polyethylene glycols (PEGs), glycols, and combinations thereof, including blends of different molecular weight PEGs optionally containing one or more glycols.

(4) Water-based Formulations: Aqueous compositions of the present invention are those in which water is present in the greatest amount, thereby forming the "vehicle." In most applications, the water-based formulation will be formed prior to use with the antimicrobial compositions of the present invention. In some applications the water-based formulations can be thickened with thickener systems. Suitable thickener systems include organic polymers or inorganic thixotropes such as silica gel, clays (such as betonite, laponite, hectorite, montmorrillonite and the like), as well as organically modified inorganic particulates materials, and the like. The thickener system can be prepared from one or more nonionic, cationic, anionic, zwitterionic, or associative polymers as long as they are compatible with the antimicrobial lipid and enhancer components of the composition. Preferably, the compositions that include an acidic enhancer component are thickened using cationic or nonionic thickeners since these perform well at low pH. In addition, many of the nonionic and cationic polymers can tolerate higher levels of salts and other additives and still maintain high viscosity.

A preferred group of nonionic polymeric thickeners include modified celluloses, guar, xanthan gum, and other natural polymers such as polysaccharides and proteins

(5) Neat Compositions: The antimicrobial lipid compositions of the present invention also may be delivered in a neat form or in a volatile solvent that rapidly evaporates to leave behind a neat composition. Such compositions may be solid, semi-solid or liquid. In the case where the compositions are solid, the antimicrobial lipid and/or the enhancer

and/or the surfactant may optionally be microencapsulated to either sustain the delivery or facilitate manufacturing a powder which is easily delivered. Alternatively, the composition can be micronized into a fine powder without the addition of other components or it may optionally contain fillers and other ingredients that facilitate powder manufacture. Suitable powders include but are not limited to calcium carbonate, calcium phosphate, various sugars, starches, cellulose derivatives, gelatin, and polymers such as polyethylene glycols.

The antimicrobial compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Delivery Methods and Devices

Formulations of the invention can be packaged into kits. Some antimicrobial lipids can be inherently reactive, especially in the presence of enhancers such as hydroxy-substituted organic acids or chelating agents. For example, the fatty acid monoesters can hydrolyze in an aqueous medium to the corresponding fatty acid, transesterify with a hydroxy-containing enhancer (e.g., lactic acid), or transesterify with a hydroxy-containing solvent. Depending on the components chosen, the antimicrobial activity of the liquid composition may be reduced and shelf life may be shortened to less than one year.

Thus, the formulations can be packaged conveniently in a two-part system (kit) to increase stability. In one example of a two-part system, all components of the formulation, except the enhancer, are present in one container, while the enhancer is present in a separate container. In another example, the first container will contain all the components of the composition, including an enhancer soluble in the propylene glycol fatty acid ester, while the second container houses a second enhancer. Contents from each container are mixed together and may be diluted before treating the applicable food or surface.

In some embodiments, the antimicrobial formulation is packaged in a single container having separate compartments for storing various components, e.g., the enhancer is in one compartment and the antimicrobial lipid, and optionally one or more surfactants, and a second enhancer are in a second compartment of the same container. Such two-compartment containers typically employ a breakable or displaceable partition between the two compartments. The partition then can be either broken or displaced to allow mixing. Alternatively, the container is configured such that a portion of the contents from each compartment can be removed, without mixing the entire contents of each compartment. See, for example, U.S. Patent Nos. 5,862,949, 6,045,254 and 6,089,389 for descriptions of two-compartment containers.

In other embodiments, a composition can be provided in two parts and the antimicrobial lipid component can be made in situ. For example, a monoglyceride could be formed in-situ from a di- or tri-glyceride in the presence of a lipase such as a mammalian or bacterially derived lipase. This may occur on the substrate or prior to application to the substrate.

In the methods of the present invention, the antimicrobial lipid compositions may be provided as a formulation suitable for delivery to a substrate. Suitable formulations can include, but are not limited to, creams, gels, foams, ointments, lotions, balms, waxes, salves, solutions, suspensions, dispersions, water in oil or oil in water emulsions, microemulsions, pastes, powders, oils, lozenges, boluses, and sprays, and the like.

Unless otherwise defined, all technical and scientific terms used herein have the same meanings as commonly understood by one of ordinary skill in the art to which this invention belongs. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting. The invention will be further described in the following examples, which do not limit the scope of the invention described in the claims.

EXAMPLES

The following examples are intended to provide further details and embodiments related to the practice of the present invention as identified in the claims. The following examples are offered for illustrative purposes to aid in understanding of the present invention and are not to be construed as limiting the scope thereof. All materials are commercially available unless otherwise stated or apparent. All parts, percentages, ratios, etc., in the examples are by weight unless otherwise indicated.

## GLOSSARY

| Material | Nomenclature in the text | Supplier |
|---|---|---|
| Glycerol monolaurate | GMLC12 | Med-Chem. Labs, MI |
| Propylene Glycol MonoCaprylate | PGMC8 | Uniqema, NJ |
| Propylene Glycol MonoCaprate | PGMC | Uniqema, NJ |
| Propylene Glycol Monolaurate | PGMC12 | Uniqema, NJ |
| Propylene glycol di-caprate | PGDC10 | Uniqema, NJ |
| Propylene glycol di-laurate | PGDC12 | Uniqema, NJ |
| Laurie Acid | Laurie Acid | Sigma Chemical Co., St. Louis, MO |
| Sodium caproyl lactylate | Pationic 122 A | RITA Chicago, IL |
| Sodium Lauroyl lactylate | Pationic 138C | RITA Chicago, IL |
| Sorbitan Monolaurate | Span 20 | Uniqema, NJ |
| 50% Dioctyl Sodium Sulfosuccinate in PEG-400 | DOSS | Cytec, NJ |
| Butylated Hydroxyanisole | BHA | EASTMAN, TN |
| Pluronic P65 Surfactant | Pluronic P65 | BASF, NJ |
| Pluronic P68 Surfactant | Pluronic P68 | BASF, NJ |
| Sorbitan monooleate | Span 80 | Uniqema, NJ |
| Sorbitan Trioleate | Span 85 | Uniqema, NJ |
| Pluronic L35 | Pluronic L35 | BASF, NJ |
| Glycerin | Glycerin | J. T. Baker, NJ |
| Lauryl/Myristyl Alcohol | Lauryl/Myristic Alcohol | Procter& Gamble, OH |
| Benzoic Acid | Benzoic Acid | Mallinckrodt, St. Louis, MO |
| Salicylic Acid | Salicylic Acid | Mallinckrodt, St. Louis, MO |
| Methylparaben | Methylparaben | Protameen Chemical, IL |
| Propylparaben | Propylparaben | Protameen Chemical, IL |
| Polyethylene Glycol 400 | PEG 400 | Dow, Midland,MI |
| Lactic Acid | Lactic Acid | PURAC, Lincolnshire, IL |
| Tartaric Acid | Tartaric Acid | Mallinckrodt, St. Louis, MO |
| Propylene Glycol | Propylene Glycol | hci, St. Paul, MN |
| Isopropyl myristate | IPM | Aldrich Chemical Co., St. Louis, MO |
| Glycerol monomyristate | MMG | Sigma Chemical Co, St. Louis, MO |
| Ethylhexylglycerin | Sensiva SC50 | Schulke & Mayi Gmbh, Germany |

Example 1

Preparation of concentrated solution

Concentrate solutions of fatty acid esters were made by one of two procedures. The first procedure consisted of weighing all components needed for a formulation as listed in Table 1 into a glass container, heating the components in an oven at 70-80°C from a few minutes to a few hours, while periodically shaking the solution by hand, until the solution was a homogenous transparent liquid.

The second procedure consisted of adding each component to a glass container while being heated on a heating plate. During heating, the solution was constantly stirred either by a magnet or a propeller stirring system. The solution was mixed until a homogenous transparent single phase liquid resulted. Table 1 lists compositions of concentrated formulations made by one of the two procedures described above. The two procedures gave equivalent solutions for further testing. All formulations had no phase separation for at least one day at room temperature. Most of the compositions were physically stable in one phase at 4°C for several months.

Table 1. Concentrated Solution Formulation

| | Concentrate Formulation | | | | | | | | | | | | |
|---|---|---|---|---|---|---|---|---|---|---|---|---|---|
| | 1 | 2 | 3 | 4 | 5 | 6 | 7 | 8 | 9 | 10 | 11 | 12 | 13 |
| GML12 | 10.0 | | | | 25.0 | 20.0 | 20.0 | 20.0 | 20.0 | 15.0 | 20.0 | 20.0 | 15.0 |
| PGMC8 | 50.0 | 70.0 | 90.0 | 45.0 | | 45.0 | | | | | 40.0 | 40.0 | 45.0 |
| PGMC10 | | | | | 40.0 | | 45.0 | | 50.0 | 40.0 | | | |
| PGMC12 | | | | 15.0 | | | | 45.0 | | | | | |
| Pationic 122A | 20.0 | | | 10.0 | 10.0 | 25.0 | 25.0 | 25.0 | | 20.0 | 10.0 | 10.0 | 25.0 |
| Pationic 138C | | | | | 10.0 | | | | | | | | |
| Span 20 | 10.0 | | | | 5.0 | 5.0 | 5.0 | 5.0 | 5.0 | 5.0 | | 15.0 | |
| DOSS | | 5.0 | 2.0 | | | | | | 20.0 | 5.0 | | | 10.0 |
| BHA | 10.0 | | | | | | | | | 5.0 | | | |
| Pluronic P65 | | 15.0 | 8.0 | 20.0 | 5.0 | 5.0 | 5.0 | 5.0 | 5.0 | 5.0 | | | |
| Span 80 | | | | 10.0 | | | | | | | 20.0 | | |
| Span 85 | | | | | | | | | | | | | 5.0 |
| Pluronic L35 | | | | | | | | | | | | 5.0 | |
| Glycerol | | | | | | | | | | | | | |
| Benzoic Acid | | 10.0 | | | | | | | | | | | |
| PEG 400 | | | | | 5.0 | | | | | 5.0 | 10.0 | 10.0 | |

Table 1 (continued) Concentrated Solution Formulation

| | High Ester Concentrate FAME Formulation | | | | | | | | | | | | | |
|---|---|---|---|---|---|---|---|---|---|---|---|---|---|---|
| | 14 | 15 | 16 | 17 | 18 | 19 | 20 | 21 | 22 | 23 | 24 | 25 | 26 | 27 |
| GML12 | | 15.0 | | 10.0 | 10.0 | | 15.0 | 15.0 | 15.0 | 15.0 | 10.0 | 10.0 | | 15.0 |
| PGMC8 | 40.0 | | 35.0 | 45.5 | 41.0 | 40.0 | | 51.0 | 45.0 | 38.0 | 45.0 | 45.0 | 34.0 | |
| PGMC10 | | 50.0 | | | | | 40.0 | | | | | | | 50.0 |
| PGMC12 | 30.0 | | 30.0 | 15.0 | 15.0 | 30.0 | | | | | | | | |

| | | | | | | | | | | | | | | |
|---|---|---|---|---|---|---|---|---|---|---|---|---|---|---|
| Lauric Acid | | | | | | | | | | 10.0 | | 10.0 | | |
| Pationic 122A | 15.0 | 25.0 | 10.0 | 24.0 | 9.0 | 15.0 | 25.0 | 20.0 | 10.0 | | | | | 20.0 |
| Pationic 138C | | | | | | | | | | | | | | |
| Span 20 | | | | 1.0 | 13.0 | | | 10.0 | 15.0 | 20.0 | 20.0 | 20.0 | | |
| DOSS | 10.0 | 10.0 | 2.0 | 2.5 | 2.5 | 5.0 | 10.0 | 2.0 | 3.0 | 5.0 | 3.0 | 3.0 | 66.0 | 10.0 |
| BHA | | | | | | | | | 2.0 | 2.0 | 2.0 | 2.0 | | |
| Pluronic P65 | 5.0 | | 13.0 | 2.0 | 9.5 | | | | | | | | | |
| Glycerol | | | | | | | | 2.0 | | | | | | |
| Lauryl/Myristyl Alcohol | | | 10.0 | | | | | | | | | | | |
| Benzoic Acid | | | | | | | | | 10.0 | 10.0 | 10.0 | | | |
| Salicylic Acid | | | | | | 10.0 | 10.0 | | | | | | | 10.0 |
| Methylparaben | | | | | | | | | | | 10.0 | | | |
| Propylparaben | | | | | | | | | | | | 10.0 | | |

To compare stability, formulations 28 and 29 provided in Table 2 were prepared according to procedures in US5460833.

Table 2 Comparative fatty acid ester solutions

| | Formulation 28 | Formulation 29 |
|---|---|---|
| GMLC12 | 1.00 | 1.00 |
| PGMC8 | 2.50 | 2.50 |
| PGMC10 | 2.50 | 2.50 |
| DOSS, 50% | 10.00 | 10.00 |
| Pluronic P68 | 5.00 | 10.00 |
| Tartaric Acid | 6.00 | |
| Lactic Acid | | 6.00 |
| Water | | 53.00 |
| Propylene Glycol | 73.00 | 15.00 |

Example 2

To evaluate the stability of the formulations, gas chromatography (GC) was used to analyze solutions for composition both initially and after aging at 50°C for 2 and 4 weeks. The solvents were all chromatography grade (EM Omnisolv®) and potassium chloride and sodium sulfate (anhydrous) were ACS reagent grade (available from Sigma Chemical Co., St. Louis, MO). The GMLC12 used as a standard was from the same lot used to make the formulations. It was determined to be 97% pure by GC assay vs. a 99+% standard of GMLC12.

Propylene glycol mono- and di-caprylate ($PGMC_8$, $PGDC_8$), propylene glycol mono- and di-caprate ($PGMC_{10}$, $PGDC_{10}$) and, propylene glycol mono- and di-laurate ($PGMC_{12}$, $PGDC_{12}$) samples for standards were obtained by distillation of the corresponding technical grade monoesters supplied from Uniqema. All were 99+% pure by GC analysis. These samples were used to generate calibration curves. Glycerol monomyristate (MMG), used as an internal standard, was 99+% pure (available from from Sigma Chemical Co., St. Louis, MO). Isopropyl myristate (IPM), was also used as an internal standard, and was obtained 98% pure (available from Aldrich Chemical Co., St. Louis, MO). The ratio of

ester to internal standard and the calibration curves allowed the analysis of formulations 14, 15, 27, 28 and 29 in Tables 1 and 2 for the composition of aged and initial samples.

Preparation of samples:

Because the concentration of analytes varied by a factor of 10 between the formulations tested, the amount of formulation assayed was adjusted so that roughly equal concentrations of active esters were present in the sample to be analyzed. This adjustment allowed one set of standard curves to be used on all formulations. Table 3 below shows the amount of sample assayed for each formulation.

Table 3 - Amount of Sample Assayed per Formulation

| Formulation | Formulation 14 | Formulation 15 | Formulation 21 | Formulation 28 | Formulation 29 |
|---|---|---|---|---|---|
| Wt. Assayed (mg) | 30 | 30 | 30 | 300 | 300 |

Triplicate samples of each were prepared. An amount within 5 mg of that specified above was added to a tarred 10 ml volumetric flask. The exact weight was recorded and the flask was brought to volume with internal standard and mixed. The first 4 formulations were assayed directly by GC. Samples of formulation 29 had to be extracted prior to GC analysis in order to remove the large amount of water present. For this, 300 mg of sample solution to be analyzed was transferred to clean 7ml vials and 0.4 wt % KCl in HPLC grade water was added. The vials were sealed and vortexed for 1 min followed by centrifugation for 5 min to form 2 phases. A portion of the lower phase was transferred to a second vial containing a small amount of $Na_2(SO_4)$, capped, and shaken briefly to remove any residual water. Aliquots were then transferred to auto sampler vials and assayed by GC. Initial and aged samples were prepared for analysis in the same manner and the concentration of active esters relative to their initial concentration is given in Table 4.

| Table 4 | Percent initial present on weight basis After aging at 50°C | | | | | |
|---|---|---|---|---|---|---|
| Active Analyzed | GMLC12 | | PGMC8 | | PGMC10 | |
| Aging time | 2weeks | 4weeks | 2weeks | 4weeks | 2weeks | 4weeks |
| Formulation 14 | NA | NA | 99.8 | 93.5 | NA | NA |
| Formulation 15 | 88.3 | 84.6 | NA | NA | 96.6 | 91.7 |
| Formulation 21 | 92.3 | 99.7 | 108 | 107 | NA | NA |
| Formulation 28 | 50.4 | 41.1 | 101 | 102 | 92.3 | 89.4 |
| Formulation 29 | 18.3 | 19.3 | 25 | 20.7 | 18.4 | 14.3 |

The results illustrated in Table 4 demonstrate that the concentrated formulations (14,15 and 21) demonstrate better shelf life than formulations 28 and 29.

Example 3

Antimicrobial efficacy of concentrated formulations in vitro

The antimicrobial efficacy of concentrated formulations in vitro at 5°C was evaluated. Several solutions were prepared by diluting concentrated formulations 16, 17 and 18 of Table 1 to a 1% active ester solution with water, lactic acid was also added to the diluted solution, as a enhancer, to give a 2% w/w lactic acid final concentration. The solution was shaken until a milky emulsion formed. The emulsion solutions were used immediately after being made.

The formulations after dilution to 1% solution in water were compared to the efficacy of a 2% lactic acid solution. The 2% lactic acid solution is commonly used as carcass disinfectant in the meat industry today.

Preparation of culture suspension:

Bacteria were grown in Tryptic Soy Broth (TSB) (available from VWR Scientific, Chicago, IL) at 35°C ± 2°C for 16-24 hours. A 0.3 ml of organism culture suspension was spread on the surface of Tryptic Soy Agar (TSA) plate that was incubated at 35°C for 16-24 hours. Bacterial cells were harvested from the agar plate with an L-rod by adding

1-3 ml of TSB and transferred to a test tube. Three bacterial strains were used: Staphylococcus aureus (ATCC# 6538), E.coli (ATCC# 11229), and non-toxic E.coli 0157: H7 (ATCC# 700728).

Test Procedure:

Diluted formulations 16, 17 and 18 in Table 1 were evaluated by aseptically transferring 20.0 ml of diluted solution into each of three Erlenmeyer test flasks containing magnetic stirring bars. Flasks were placed in a controlled 5°C water bath equipped with stirring capacity. Magnetic stirrers were adjusted so the flasks are stirred rapidly without splashing. A portion (0.1 ml) of the culture suspension was added to each of the flasks. The exposure time consisted of 2 min, 5 min, 10 min and 1 hr. At the end of each exposure time, 1.0 ml of inoculated sample was transferred from each of the flasks into tubes containing 9.0 ml of Letheen broth and then vortexed. This is the $10^{-1}$ dilution. After vortexing, the solution was ten-fold sequentially further diluted by transferring 1 mil into 9 ml the letheen broth (which also neutralizes the FAME antimicrobial reaction). From each of the dilutions, 0.1 ml volume was plated onto a TSA plate and spread with L-rod. Plates were incubated at 37°C for 24 hrs and colony forming units were counted. Tests were performed with three replicates of germicidal solution. The diluted bacterial suspensions were plated in duplicate.

For the initial inoculum plates of each organism, the colony forming units (CFU) were counted on the dilution level that had counts between 25-250. The average of the two duplicate plates at the selected dilution level was used. The initial inoculum count was calculated using the following formula:

$$\text{Initial inoculum count} = T_{time=0} = \text{Average CFU of 2 replicates x [dilution level] x 0.005}$$

(Since the sample inoculums were diluted (0.1 ml in 20.1 ml FAME)

For the test plates of each organism at each time period, the CFU's were counted on all the $10^{-2}$ and $10^{-3}$ plates. The dilution level that has counts between 25-250 was determined and used. The average of three duplicate plates at the selected dilution level were used to calculate the test plate count at the given time using the following formula:

$$T_{time=x} = \text{Average CFU of 3 replicates at given time x [dilution level ]}$$

Average plate count of 3 replicates at exposure time point.

The log reduction was determined by taking the logarithm of $T_{time=x}$.and T time=0 and using the following formulas to determine log reduction:

$$\text{Log reduction at x time point} = \log T_{time=0} - \log T_{time=x}$$

The results from in vitro testing of the diluted high concentrate FAME solution compared to 2% lactic acid are in Table 5. Testing temperature was between 5° to 8°C.

| TABLE 5 In Vitro Testing- Log Reduction | | | | |
|---|---|---|---|---|
| Time | 2% LA Only | Formulation 16 | Formulation 17 | Formulation 18 |
| E. coli ATCC 11229, initial inoculum counts 8.3 LOG | | | | |
| 2 min | <2.72 | 4.41 | 5.89 | 6.22 |
| 5 min | <2.72 | 5.93 | 6.25 | 6.31 |
| 10 min | <2.72 | 6.33 | 6.31 | 6.31 |
| 1 hr | <2.72 | | | |
| E. coli O157:H7 ATCC 7007728, initial inoculum count 8.36 LOG | | | | |
| 2min | | | >6.36 | |
| 5 min | | | >6.36 | |

(continued)

| TABLE 5 In Vitro Testing- Log Reduction | | | | |
|---|---|---|---|---|
| Time | 2% LA Only | Formulation 16 | Formulation 17 | Formulation 18 |
| 10 min | | | >6.36 | |
| Staph. aureus, ATCC 25923, initial inoculum count 8.06 LOG | | | | |
| 2 min | <2.72 | 5.54 | 5.96 | 5.23 |
| 5 min | <2.72 | 4.65 | 6.33 | 5.97 |
| 10 min | <2.72 | 6.37 | 6.43 | 6.13 |
| 1hr | <2.72 | | | |

The results demonstrate that concentrated solution diluted to 1% (1:100 dilution in water), had a much higher antimicrobial efficacy than lactic acid alone at refrigeration temperatures.

Example 4

Preparation of culture cocktail suspension

Bacteria were grown in the same procedure described in Example 3. Bacteria strains used were four Salmonella isolates (*S. enteritidis* phage type IV, *S. typhimurium* (ATCC 13311), NATO 32091 (obtained from Cargill Inc., Wayzata, MN), FRB 93922 (obtained from Cargill Inc., Wayzata, MN); and *E.coli* (ATCC 11229). To prepare the cocktail, add 2 mls of E. coli, and 0.5 ml of each of the four Salmonella cultures. Shake well. Run an inoculum count on the bacterial cocktail.

Inoculatation of meat pieces with bacterial inoculum cocktail

Several meat pieces (5 x 5 x 0.6 cm in size) were inoculated. The lean and fat samples (from a retail meat supplier or commercial slaughterhouse) were placed on a 8 x 11 tray and inoculated with the inoculum cocktail by spraying 1 stroke of cocktail solution on to the meat pieces from a hand pumped spray bottle sufficient to wet the surface completely. The tray of meat samples was placed in 40°C oven for 20 minutes.

Determination of inoculated meat bacterial count

Three inoculated meat samples were each placed in a Whirlpak bag (available from VWR Scientific, Chicago, IL) to which 99 ml. of Butterfields Buffer (available from International Bio Products, Bothell, WA) was added. The bags were stomached for 30 sec. to assist with removal of bacteria from meat. An aliquot (11 ml.) was removed from each sample bag and another 99 ml. Butterfields Buffer was added, mixed thoroughly to give a solution for further testing. Petrifilm™ E. coli/Coliform count plate (available from 3M, St. Paul, MN), Salmonella XLD agar (available from Remal, Inc., Lenexas, KS) and Petrifilm™ Aerobic Count (available from 3M, St. Paul, MN) were used as media with serial ten-fold dilutions using Butterfield buffer. Plates were incubated for 18-24 hours at 37°C after which time they were counted as described in Example 3 to give an initial bacteria count.

Formulation Treatment and Determination of Bacterial Log Reduction

Formulations 1 and 7 from Table 1 were diluted to 1% in water before use. Lactic acid was also added to the diluted solution, as enhancer, to a 1% or 2% final w/w concentration. Solution was shaken well until a milky emulsion formed. The emulsion solutions were used immediately after being made.

For each solution treatment, 6 inoculated meat samples were dipped in the prepared solution at the designated temperature for 5 minutes. All 6 meat samples were removed from the solution at the designated temperature, the excess solution was allowed to drip off from meat pieces for a few seconds, and the meat samples were then placed in Whirlpak bags. Three samples were used immediately for 5-minute counts and three samples were placed in a 6-8°C refrigerator for 24 hours to provide the 24 hour count samples. At the designated times (5 min, 24 hours.) 99 ml. of Butterfields Buffer was added to the samples in the Whirlpak bags. The samples in bags were massaged by hand for 30 sec. to assist with removal of bacteria from meat. An 11 ml. aliquot was removed from the bag and combined with another 99

ml. of Butterfields Buffer, after thorough mixing the solution was used for bacterial count testing. Petrifilm™ E. coli/ Coliform count plate (available from 3M, St. Paul, MN), Salmonella XLD agar and Petrifilm™ Aerobic Count (available from 3M, St. Paul, MN), were used as the media with serial ten-fold dilutions using Butterfield buffer. Plates were incubated for 18-24 hours at 37°C. The above steps were repeated using 2% lactic acid and water as a reference to compare with the formulation solutions.

Plates were read after incubation and log reductions determined as indicated in Example 3. The results are provided in Tables 6 - 8.

Table 6. Log reductions on beef lean side

| | Treatment Temperature | E.Coli Initial inoculum 5.5 logs | | Salmonella Cocktail Initial inoculum 5.5 logs | | Total Aerobic Count Initial inoculum count 6.2 logs | |
|---|---|---|---|---|---|---|---|
| | | 5 min dip[1] | 24 hr storage at 5 deg C[2] | 5 min dip[1] | 24 hr storage at 5 deg C[2] | 5 min dip[1] | 24 hr storage at 5 deg C[2] |
| Formulation 7 | 23°C | 4.82 | 2.57 | 4.32 | 2.37 | 2.70 | 1.91 |
| Formulation 1 | | 3.45 | 3.08 | 3.48 | 2.82 | 1.76 | 1.44 |
| 2% Lactic Acid | | 2.83 | 1.82 | 1.62 | 1.77 | 0.97 | 0.82 |
| Formulation 7 | 40°C | 4.85 | 3.30 | 4.80 | 4.80 | 3.04 | 2.68 |
| Formulation 1 | | 4.65 | 2.78 | 4.03 | 3.14 | 2.54 | 2.22 |
| Formulation 7 | 50°C | 4.81 | 4.21 | 4.79 | 4.59 | 3.47 | 3.16 |
| Formulation 1 | | 4.51 | 3.25 | 4.79 | 4.36 | 3.56 | 2.39 |

[1]Data at "5 min dip" means that meat samples were tested immediately after 5 min dipping. [2]Data at "24 hr storage at 5°C" means that meat samples dipped for 5 minutes and then stored 24 hours at 5°C before testing.

Table 7. Log reduction of bacteria on beef lean side

| % Formulation 1 in water[3] (Dilution levels) with water at 23°C | E.Coli initial inoculum 5 logs | | Salmonella cocktail initial inoculum 5.5logs | |
|---|---|---|---|---|
| | 5 min dip[1] | After 24 hrs stored at 5°C[2] | 5 min dip[1] | After 24 hrs stored at 5°C[2] |
| 1.0% (1:100) | 3.45 | 3.08 | 3.48 | 2.82 |
| 0.5% (1:200) | 2.86 | 3.13 | 2.38 | 2.45 |
| 0.33% (1:300) | 3.35 | 2.64 | 3.25 | 2.48 |
| 2% Lactic Acid only | 2.83 | 1.82 | 1.62 | 1.77 |

[1]Data at "5 min dip" means that meat samples were tested immediately after 5 min dipping. [2]Data at "24 hr storage at 5°C" means that meat samples dipped for 5 minutes and then stored 24 hours at 5°C before testing
[3]Lactic acid was 2% w/w as final concentration

Table 8 Log reduction of bacteria on beef fat side

| FAME formulation 1diluted to 1% and treatment at 23°C for 5 min. | Salmonella Cocktail Initial inoculum count 5.98 Logs | | E.Coli Initial inoculum count 5.4 Logs | | Total Aerobic Count Initial inoculum count 6.62 Logs | |
|---|---|---|---|---|---|---|
| Enhancer Lactic Acid Level | 1% w/w | 2% w/w | 1% w/w | 2% w/w | 1% w/w | 2% w/w |
| Log reduction after 5 min dip | 2.53 | 5.28 | 3.55 | 4.7 | 1.78 | 5.51 |
| Log reduction after 24 hrs stored at 5°C | 3.39 | 5.28 | 3.54 | 4.7 | 1.93 | 5.17 |
| [1]Data at "5 min dip" means that meat samples were tested immediately after 5 min dipping. [2]Data at "24 hr storage at 5°C" means that meat samples dipped for 5 minutes and then stored 24 hours at 5°C before testing | | | | | | |

The results in tables 6, 7 and 8 demonstrate the antimicrobial efficacy of concentrated solutions (diluted before use) relative to that of 2% lactic acid alone. The data in Table 6 also shows that high temperature in general improves the FAME formulation efficacy.

Example 5

<u>Preparation and Inoculatation of meat pieces with bacterial inoculum</u>

Bacteria were grown in the same procedure described in Example 3. The bacteria strain was non-toxic E.coli 0157: H7 (ATCC# 700728).

Several refrigerated adipose beef pieces approximately 100 cm2 x 0.3 cm in size were inoculated using a procedure similar to that described in example 4. After inoculation, the meat pieces were placed in a cooler at 5°C for 30 +/- 5 minutes to allow attachment of the organisms.

<u>Initial sample inoculum determination</u>

A 11.4 $cm^2$ coring device was used to cut surface tissue excisions no thicker than 3 mm from the inoculated samples. The stainless steel coring device was disinfected prior to each run to prevent cross-contamination among the samples. Each circular tissue excision was placed in a filter stomacher bag with 15 ml of letheen diluent and stomached for 1 minuted prior to plating. Plating was done on PetrifilmTM Enterobacteriaceae plates (Petrifilm EB, available from 3M, St. Paul, MN) using the undiluted sample from the stomacher bag. A series of ten-fold sequential dilutions were made and plated on Petrifilm EB. The plates were incubated and counted as recommended on the package insert.

<u>Application of Treatments:</u>

A 11.4 $cm^2$ coring device was used to cut surface tissue excisions no thicker than 3 mm from the inoculated samples. The stainless steel coring device was disinfected prior to each run to prevent cross-contamination among the meat samples.

Formulations 16 and 17 from Table I were diluted to 1% w/w in water. Lactic acid was also added to the diluted solution, as enhancer, to a final 2% w/w concentration. The solution was shaken well until a milky emulsion formed. The emulsion solutions were used immediately after being made.

The inoculated circles were treated with the diluted solution or with 2% lactic acid only solution (control sample) by immersing them in the solution for 5 min. at 5 deg C, 23 deg C, and 50 deg C. The circles were removed immediately

and the excess solution was allowed to drip from the circle for 5 sec.

The circles were placed in stomacher bags. Two of them were placed into the refrigerator and the third was tested by adding 15 ml of Letheen diluent to the third stomacher bag. This bag was stomached for 1 minute prior to plating. Duplicate samples were plated from the undiluted sample in the stomacher bag. Serial ten-fold dilutions were made and plated on a Petrifilm EB plate from each dilution. After incubation the plates were counted as recommended on the package insert. After 1 h refrigeration, one of the refrigerated stomacher bags was removed and the above steps repeated for the 1 h test circle. After 24 h refrigeration, the last of the stomacher bags was removed and the above steps repeated for the 24 h test circle.

Plates were read after incubation and log reductions determined as indicated in Example 3. The results presented in Table 9.

Table 9. Log reduction on Beef Fat.

| E. coli 0157:H7, initial inoculum | 5.55 logs | | | 5.11 logs | | | 4.98 logs | | |
|---|---|---|---|---|---|---|---|---|---|
| Log Reduction after beef adipose treated with diluted formulations[1] | | | | | | | | | |
| | Formulation 16 | | | Formulation 17 | | | 2% lactic acid alone | | |
| | 5°C | 23°C | 50°C | 5°C | 23°C | 50°C | 5°C | 23°C | 50°C |
| 5 min dip[2] | 0.77 | 1.22 | 1.30 | 0.74 | 1.13 | 1.81 | 0.51 | 0.87 | 1.72 |
| 60 min at 5°C[3] | 1.25 | 1.68 | 1.81 | 1.13 | 1.65 | 2.10 | 1.37 | 1.74 | 2.43 |
| 24 hours at 5°C[4] | 2.77 | 3.26 | 4.18 | 2.44 | 3.57 | 4.08 | 2.26 | 2.71 | 2.54 |

[1]-Treatment with diluted formulations at various solution temperatures. Fat tissue is maintained at 5°C.
[2]Data at "5 minutes dip" means that the fat samples were tested immediately after 5-minute dip treatment
[3]Data at "60 min at 5°C" means that fat samples were tested after storage at 5 degC for 60 minutes after the 5 min. treatment.
[4]Data at "24 hours at 5°C" means that fat samples were tested after storage at 5degC for 24 hours after the 5 min. treatment

The diluted formulations demonstrated better antimicrobial efficacy as compared to 2% lactic acid alone on beef fat surfaces. These data also show that solution efficacy increases with time showing a residual effect of the formulations on meat. The positive effects of increasing temperature are also demonstrated by the data.

Example 6

Preparation of culture suspension and inoculatation of ground meat samples

A Salmonella bacteria cocktail was grown using the same procedure described in Example 3 using four Salmonella isolates: *S. enteritidis* phage type IV, *S. typhimurium* (ATCC 13311), NATO 32091 (obtained from Cargill Inc., Wayzata, MN), FRB 93922 (obtained from Cargill Inc., Wayzata, MN). The cocktail was prepared by adding 0.5 ml of each of the four Salmonella cultures and mixing well by shaking.

The ground beef was inoculated by adding a measured weight of ground beef and Salmonella cocktail inoculum into a KitchenAid™ mixer equipped with a paddle mixer. The sample was mixed 1 min. After mixing the inoculated ground beef was placed in a cooler at 5°C for 10 min to allow attachment of the organisms.

Inoculated Sample initial inoculum determination

Inoculated 11-g aliquots of ground beef were placed in separate filter stomacher bags with 99 ml of letheen diluent in each, stomached for 1 minute. Serial ten-fold sequential dilutions were made with letheen broth. Samples were plated on Petrifilm™ E. coli/Coliform count plate (available from 3M, St. Paul, MN), Petrifilm™ Aerobic Count (available from 3M, St. Paul, MN) and Salmonella XLD agar plates for salmonella cocktail inoculated ground beef. Petrifilm plates were incubated for 24 +/-2 h at 35°C and counted as recommended on the package insert. The XLD plates were incubated overnight at 35°C and the black colonies were counted as presumptive Salmonella.

Application and Testing:

Formulation 22 was diluted in water; lactic acid was also added to the water.

The diluted solution contains 35% formulation 22, 40% lactic acid, The solution was shaken well until a milky emulsion formed; the solution was used immediately after being made.

Weighed amounts of inoculated ground beef were added into the KitchenAid™ mixer equipped with a paddle mixing head. The diluted solution based on formulation 22 was placed in a pressure pot (23°C) connected to a spray nozzle. The solution was sprayed into the inoculated ground beef(5°C) contained in the KitchenAid™ while mixing occurred with the paddle mixer. The sprayed meat contained about 5% w/w aqueous solution, with 1% FAME and 2% lactic acid. The spray was delivered to the ground beef in 15 sec.(total spraying time), with a total mixing time of 3 minutes. The treated ground beef was placed in the refrigerator again. At each desired time point, an 11-g aliquot of ground beef was weighed out. The same inoculum determination process as described above was used.

Plates with counts that are within the counting range of the plate, i.e. between 15-100 cfu per plate were used for further analysis. The results were converted to log 10 and the replicates averaged. The results of the treated samples were subtracted from the results of the analogous untreated samples to determine the log reduction of the treatment.

Table 10 contains the results for Log reduction of bacteria in ground beef treated with diluted formulation 22, using the application procedure described above.

| Table 10 Log reduction in Inoculated Ground Beef | | |
|---|---|---|
| Storage at 5°C | Salmonella Cocktail (Initial inoculum count 5.10 Logs) | Total Aerobic Count (Initial inoculum count 6.32 Logs) |
| 10 Min | 1.77 | 2.67 |
| 1 Hour | 3.19 | 2.90 |
| 24 Hour | 5.10 | 6.22 |

Table 11 contains the results for log reduction of native bacteria in uninoculated ground beef. The application is the same as described above, except that the diluted formulation 22 was pipetted, not sprayed, into the ground beef, and the ground beef was not inoculated.

| Table 11 Log reduction of native bacteria in uninoculated ground beef | | | | | | |
|---|---|---|---|---|---|---|
| | Total Aerobic Count | | | Enterobacteriaceae Count | | |
| Storage at 5°C | Log average of untreated ground beef | Log average of treated ground beef | LOG reduction | Log average of untreated ground beef | Log average of treated ground beef | LOG reduction |
| 10 Min | 4.54 | 3.24 | 1.30 | 0.77 | 0.00 | 0.77 |
| 1 Hour | 4.50 | 2.89 | 1.61 | 0.43 | 0.00 | 0.43 |
| 24 Hour | 5.10 | 0.60 | 4.50 | 1.00 | 0.00 | 1.00 |
| 48 Hour | 4.97 | 0.00 | 4.97 | 1.80 | 0.00 | 1.80 |

Results in tables 10 and 11 show the antimicrobial efficacy of diluted formulations. At 5°C, after 48 hrs, the inoculated meat and un-inoculated meat had undetectable bacteria left in ground beef, using the testing method described above. The 24 hours and 48 hours kill data are surprisingly high, which demonstrate again the residual killing effect of the formulations.

Example 7

The testing procedure was used similar to that described in Example 6 except that ground beef was inoculated in a bag mixed by hand for about 2 min., and formulation 26 was directly added to the meat to give a 1% solution w/w of meat, followed by 2% lactic acid. Then the ground beef sample was mixed by hand for about 2 min.

Table 13 contains the Log reduction results for treating ground beef concentrated formulations.

| Table 13 Log reduction with Formulation 26 | | |
|---|---|---|
| Storage at 5°C | Total Aerobic Count (Initial inoculum count 3.84 logs) | Salmonella Cocktail (Initial inoculum count 3.63 logs) |
| 10 Min | 0.76 | 1.37 |
| 1 Hour | 0.96 | 1.69 |
| 24 Hour | 1.27 | 2.42 |

Formulation 26 inactivated almost 2.5 logs of Salmonella cocktail at 5°C in 24 hours, proving the antimicrobial effectiveness of a concentrated solution.

Example 7A

Undercooked Ground Beef

The USDA recommends that ground beef be cooked to 165 def F (66 deg C) to kill any pathogenic bacteria present in the meat. The formulations of the present invention demonstrate increased efficacy with increased temperatures. Adding the formulations of the present invention to ground beef can reduce the risk of residual human pathogens if the hamburger is heated to some temperature less than 66 deg C which results in the ground beef being undercooked as demonstrated by the following example.

Preparation of culture suspension and inoculatation of ground meat samples

E.coli 0157: H7 (ATCC# 700728) cultures were prepared as in Example 3. The inoculum had a working population of $10^8$ organisms/mL as determined by optical density using a spectrophotomer set at 600nm. The ground beef was inoculated by adding 497 grams of ground beef and 10 mL of the E.coli 0157: H7 inoculum into a KichenAid™ mixer equipped with a paddle mixer. The sample was mixed 1 min. After mixing the inoculated ground beef was placed in a cooler at 5°C for approximately 10 min to allow attachment of the organisms.

Treatment of Ground Beef

Formulation 30 was prepared as in example 1. Formulation 30 contained 10% GML, 50% PGMC8, 20% Pationic 122A, 10% SPAN 20, and 10% PGMC12 by weight. The enhancer was made by diluting lactic acid to 25% in deionized water.

The inoculated ground beef (507 g) was removed from the cooler, and formulation 30 and the enhancer were added separately using a pressurized sprayer (Sprayer Systems Co., Wheaton, IL) with a fan nozzle while the mixer blended the combination with the paddle attachment for 3 minutes total at a low mixing setting. Enhancer was delivered first at a spray rate of 30mL/min during the first 1.5 minutes of mixing and then Formulation 30 was delivered at a spray rate of 7.5ml/min while the mixing continued for an additional 1.5 minutes. Sufficient enhancer and FAME formulation were added to give an additional 5% weight to the mixed mass with 1% coming from formulation 30, 1%from the enhancer, and 3% water.

Three 113.5 g samples of treated beef were weighed out, made into a ball that was then formed into a patty using a Tupperware hamburger press. The resulting patties were uniform, approximately 12 mm in thickness. The patties were cooked on an electric skillet (11" West Bend, Model#72108) preheated for five minutes at a setting of 350. The three patties were placed equal distance apart in a triangular formation on the skillet with each patty centered over the heating coil of the skillet. Each side was heated 2 minutes before turning over, until the desired internal temperature was achieved. The temperature was measured using a digital thermometer (VWR Scientific Products, Model #23609-160) placed to read the temperature in the center of the patty.

The three patties were removed from the skillet when the temperature reached 60 C (140 degrees F, approximately 4.2 minutes). The patties were placed on a sterile foil paper, cut into 4 quarters and an 11 g sample was removed from the center of each quarter for a total of 4 samples per patty. The 11 g samples were placed in 3M Stomacher bags with 99 mL of sterile letheen broth, stomached for 1 minute, and then diluted as described in Example 6. Plating was done on Petrifilm™ E. coli/Coliform count plate (available from 3M, St. Paul, MN), Petrifilm™ Aerobic Count (available from 3M. St. Paul, MN) plates with the analysis as in Example 6 to calculate the log population counts.

Plates with counts that are within the counting range of the plate, i.e. between 15-100 cfu per plate were used for further analysis. The results were converted to log 10 and the replicates averaged.

The above procedure was repeated to generate another three FAME treated patties that were removed once they reached 66 C (150 degrees F, approximately 6 minutes). For comparison 3 batches of inoculated ground beef were prepared as above without adding Formulation 30. Three ground beef patties were prepared at each of 3 temperatures: 60 C, 66 C, and 74 C (165 degrees F) using the same batches of inoculated ground beef.

Table 14 contains the results for bacteria level in ground beef on heating after inoculation with *E.coli* 0157: H7.

| Table 14. Log count of population in Ground Beef | | | | | | |
|---|---|---|---|---|---|---|
| | Enterobacteriaceae Count | | | Aerobic Count | | |
| Cooking Temperature | No FAME | FAME Treated | Log Reduction | No FAME | FAME Treated | Log Reduction |
| 60 C | 5.05 | 2.19 | 2.86 | 5.16 | 2.55 | 2.61 |
| 66 C | 3.81 | 0.05 | 3.76 | 4.08 | 0.31 | 3.77 |
| 74 C | 0.151 | Not tested | | 0.23 | Not tested | |

Example 8

Formulation 15 was evaluated on oranges inoculated with E. coli cocktail. Formulation 15 was diluted to 1% w/w in water. Lactic acid was also added to the diluted solution, as enhancer, to a final 2% w/w concentration. The solution was shaken well until a milky emulsion formed. The emulsion solutions were used immediately after being made.

Inoculation of Oranges:

An E. coli cocktail was prepared using four E. coli strains (ATCC # 25922, ATCC #11229, ATCC# 35218, and CREC isolate 97-1) and placed into the nutrient

broth. The broth was incubated 20-24 hours at 35°C. This gave an inoculum of about $10^9$ organisms/ml. Oranges were obtained from the local grocery, the diameter was measured and the surface area calculated assuming a sphere. The average orange surface area was 66.70 $cm^2$. Wax was removed by washing with mild detergent (Ivory), rinsing with DI water and allowing the orange to air dry overnight.

Two methods were used to inoculate the oranges:

Method A: Oranges were added to an inoculated Nutrient Broth. Oranges were kept beneath the surface by a weighted clean container. After 15 minutes the oranges were removed and allowed to air-dry for one hour.
Method B: 10 spots were inoculated on the orange with 20ml of the organism (cocktail) and the inoculum was allowed to air-dry for 1 hour.
After the inoculated oranges had dried, 5 oranges were placed in a ziplock bag along with 500 ml of chilled 0.1% peptone water (made from dehydrated media available from VWR Scientific, Chicago, IL), and placed on ice in a reciprocal shaker for one hour. These oranges served as the control to determine the maximum recovery of organisms per $cm^2$ of inoculated orange surface. This procedure was repeated with two more batches of five oranges each to give results in triplicate.

The same inoculation procedure was performed for testing a Salmonella cocktail using Salmonella Typhimurium (ATCC #14028), Salm. Mbandaka (ATCC#51958), Salm. Muenchen (ATCC#8388), and Salm. Montevideo (ATCC#8387).

Treatment and Analysis:

Diluted formulation 15 was heated and maintained at 40°C. Five oranges were added to the solution and soaked for 30 seconds, 1 minute, and 2 minutes. The oranges were stirred occasionally with a clean spoon. The oranges were removed to a sterile beaker containing sterile distilled water for 10-15 seconds to remove excess treatment. Without drying the oranges were then placed in a ziplock bag with 500 ml of 0.1 % peptone water and placed on ice in a reciprocal shaker for 1 hour. Samples were run in triplicate. After 1 hour on the shaker, the pH of the sample was checked and then solution was pipetted onto Petrifilm E.coli/Coliform plates or into dilution bottles for further serial ten-fold dilutions. Samples were plated in duplicate.

The average surface area of the oranges in $cm^2$ was calculated using the average diameter measured. A conversion factor was determined by dividing the number of ml of buffer used (500 ml) by the total orange average

surface area. This conversion factor was used to covert the actual colony counts to colonies per square centimeter ($cm^2$) of orange. Multiplying the actual CFU/ml from each plate by this conversion factor was used to obtain a count of CFU per square cm.

Table 15 contains the results for log reduction of E.coli cocktail on an orange surface after treatment with diluted Formulation 15 at 40°C. The initial inoculum of E.coli cocktail was 5.13 logs

| Table 15 Log reduction of E.coli cocktail on orange surface | | | | | | |
|---|---|---|---|---|---|---|
| | E.coli cocktail, Initial inoculum 5.13Log | | | Salmonella cocktail Initial inoculum 5.51 Log | | |
| Time | 30 sec | 1 min | 2 min | 30 sec | 1 min | 2 min |
| Formulation 15 | 4.30 | 5.13 | 4.49 | 3.08 | 2.12 | 4.17 |
| Water | 1.04 | 1.20 | 1.16 | 0.98 | 0.74 | 1.49 |

Example 9

Antifungal efficacy on oranges

The test method and sample preparation were the same as that described in Example 8 except that two fungi were inoculated separately to the oranges surface: Penicillium italicum (ATCC# 32079) and Penicillium digitatum (ATCC# 34644). Both Formulations 15 and 27 was diluted to 1%. Lactic acid was added to formulation 15 only to 1%. No addition of lactic acid was made to formulation 27.

Table 16 contains the results for log reductions of two fungi on orange surface after treatment with diluted formulations at 50°C.

| Table 16 Log reductions of two fungi on orange surface | | | | | | |
|---|---|---|---|---|---|---|
| Treatment Time | Penicillum italicum (Initial Inoculum 4.08 logs) | | | Penicillum digitatum (Initial inoculum 3.17 logs) | | |
| | Formulation 15 | Formulation 27 | Water | Formulation 15 | Formulation 27 | Water |
| 30 seconds | 1.27 | 1.67 | 0.58 | 2.46 | 2.20 | 1.23 |
| 1 minute | 0.84 | 0.90 | -0.07 | 0.95 | 2.59 | 1.43 |
| 2 minute | 1.28 | 2.04 | 0.14 | 1.81 | 2.38 | 1.12 |

Note that the formulation difference between formulation 15 and 27 was the enhancer. Formulation 15 used 1% lactic acid as enhancer and Formulation 27 used ester soluble salicylic acid as enhancer at 0.1% level after the dilution.

Example 10

Efficacy on nonwoven polypropylene

Example 10 tests the formulations for use as antimicrobial coatings, which make textiles resistant to bacteria attack. The test method is based on AATCC Test Method 100-1993, Antibacterial Finishes on Textile Materials: Assessment, with some modifications: One and a half-inch squares of nonwoven polyproylene were inoculated and stored in petri dishes instead of glass jars. The number of swatches used was dependent on the material type; materials were not sterilized; and dilutions of test organisms were made in tryptic soy broth (TSB) instead of nutrient broth. Incubation periods were one and twenty-four hours. The material swatches were kept in petri dishes. Samples were dispensed onto tryptic soy agar (TSA) instead of nutrient agar. The challenge bacteria used were Staphylococcus aureus (ATCC# 6538) and K. pneumonia (ATCC #23357).

A brief description of the test procedure used is as follows. Formulations 14, 15, 19 and 20 were diluted to 1% w/w in water. Lactic acid was also added to the diluted solution, as enhancer, to a final 1% w/w concentration. The solution was shaken well until a milky emulsion formed. These emulsion solutions were used immediately.

Samples were placed into the diluted formulation for 10 seconds. They were then removed and dried for 18-24 hours. The treated textile samples were inoculated with 1 ml of bacteria being dispensed in the center of the textile. Multiple treated squares of textile samples were provided per inoculation to absorb the total 1 ml inoculum. The inoculated textile samples were placed into a petri dish. At time 0, 1 hour and 24 hours, textile samples were removed and put into

letheen broth, shaken well and serial ten-fold dilution was done. The dilution solution was plated in duplicate in TSA and the bacteria counts were enumerated. The initial inoculum counts were compared to those after treatment with the bacteria reduction is reported in percentage reduction in Table 17.

Table 17 Percentage reduction on textile

| (a) reduction of Staph. aureus | | |
|---|---|---|
| Formulation | 1 hour | 24 hours |
| Formulation 20 | 100 | 100 |
| Formulation 19 | 100 | 100 |
| Formulation 14 | 99.6 | 100 |
| (b) reduction of K. pneumonia | | |
| Formulation | 1 hour | 24 hours |
| Formulation 15 | 75.19 | 97.22 |
| Formulation 14 | 83.96 | 100 |

Results in tables 17 a and b demonstrated that the formulations of this invention were effective at reducing levels of bacteria on textile samples.

Example 11.

Antimicrobial efficacy on hard surfaces

Formulation 1 was evaluated to disinfect hard, inanimate surfaces such as stainless steel. Formulation 1 was diluted to 2% w/w in water. Lactic acid was also added to the diluted solution, as enhancer, to a final 2% w/w concentration. The solution was shaken well until a milky emulsion formed. The emulsion solutions were used immediately after being made.

Inoculum and testing procedure:

The procedure from AOAC Official Methods (AOAC Official Method 991.47, Testing Disinfectants against Salmonella Choleraesuis and AOAC Official Method 955.15: Testing Disinfectants against Staphylococcus aureus) was used for testing disinfectants against the following organisms: Staphylococcus aureus (ATCC# 6538); Salmonella Choleraesuis (ATCC# 10708); and E.coli (ATCC# 11229). Initial Inoculum: S.aureus 6.33 logs, E.coli: 6.98 logs; S. Choleraesuis: 8.35 logs.

Briefly, in this test, hollow stainless steel or glass cylinders (Penicylinders) are coated with the challenge bacteria. The bacteria are allowed to dry on the penicylinders for a set period of time. The penicylinders with the dried bacteria inoculum are dipped into the diluted formulation for 10 minutes, removed and placed into neutralizer solution (letheen broth) for 30 seconds and then put into TSB for 24 hours. At the end of 24 hours the tubes containing the penicylinders are checked for turbidity and scored as either growth or no growth.

Treated inoculated surfaces showed no growth for 10 out of 10 test pieces for the each of the 3 different bacteria tested (Staphylococcus aureus (ATCC# 6538); Salmonella Choleraesuis (ATCC# 10708); E.coli (ATCC# 11229)). The results indicate that the diluted high ester concentrate Fame formulations are highly efficient hard surface disinfectants.

Example 12

Sporicidal efficacy against Fungal spores on oranges

Formulation 15 and 27 was diluted to 10% and 1% in water. Lactic acid was added to formulation 15 only to 2%. No lactic acid was added to diluted formulation 27.

The kill rate assay procedure was very similar to that described in Example 3, with the following changes: Penicillium italicum ATCC# 32079 and Penicillium digitatum ATCC# 34644 spores were prepared as the challenge organisms. Potato Dextrose was used as media instead of TSA. FAME formulation treatment was performed at 50°C, not 8°C.

Table 18 contains the results from In Vitro testing showing log reductions for two fungal Spores after treatment with the formulations at 50°C.

| Table 18 In Vitro treatment of fungal spores at 50°C. | | | | | | | | | | |
|---|---|---|---|---|---|---|---|---|---|---|
| | Penicillum italicum ATCC# 32079 Initial Inoculum 6.57 logs | | | | | Penicillum digitatum ATCC# 34644 Initial Inoculum 5.65 logs | | | | |
| Treatment Time | Formulation 15 2% lactic Acid as enhancer | | Formulation 27 Salicylic acid as enhancer | | Water | Formulation 15 2% lactic Acid as enhancer | | Formulation 27 Salicylic acid as enhancer | | Water |
| | 10% in water | 1 % in water | 10% in water | 1% in water | only | 10% in water | 1 % in water | 10% in water | 1 % in water | only |
| 2 minute | 4.33 | 4.57 | 4.12 | 3.65 | 0.86 | 3.04 | 3.65 | 3.65 | 3.65 | 0.22 |
| 5 minute | 4.57 | 4.57 | 4.46 | 3.65 | 0.86 | 3.65 | 3.65 | 3.65 | 3.65 | 0.65 |
| 10 minute | 4.57 | 4.57 | 4.51 | 3.65 | 0.86 | 3.65 | 3.47 | 3.65 | 3.65 | 1.88 |

Example 13

Into a heated 4 oz glass jar on a heating plate, add in sequentially 40 grams of Ethylhexylglycerin, 10g of glycerol monolaurate, 10 grams of Pationic 122A, 20 Grams of Span 20, 5 grams of DOSS, and finally 10 grams of IPA. The solution was constantly stirred by a magnet bar until the solution turn to a homogenous transparent single phase liquid, and then cooled to room temperature.

Example 14

Method of Creating Treated Ground Beef

Inoculum preparation

Solutions of bacteria were prepared from *Escherichia coli* ATCC 11229 and pathogenic *Escherichia coli* O157: H7 cocktail (ATCC 35150, ATCC 43894, ATCC 43895). All strains were grown in individual tubes containing 10 mL of tryptic soy broth (TSB) at 37°C for approximately 24 hours.

The *E. coli* 11229 inoculum solution was prepared by transferring two 10 mL inoculated TSB samples into a plastic spray bottle. The inoculum was then diluted by the addition of two 90 mL portions of sterile Letheen broth to obtain a solution with a working population of $10^8$ colony forming units(CFU)/milliliter(mL)..

The *E. coli* O157:H7 cocktail solution was prepared by adding two 10 mL inoculum samples made from equal parts (3.3 mL) of the individual organisms (ATCC 35150, ATCC 43894, ATCC 43895) into a plastic spray bottle and, diluting this inoculum cocktail by the addition of two 90 mL portions of sterile Letheen broth to obtain a working population of $10^8$ CFU/mL.

<u>Meat preparation and inoculation</u>

Cold (~5°C) beef trim meat( boneless beef arm roast) obtained from grocery store, with an approximate fat content of 10%-15%, was cut lengthwise into linch (2.54cm) wide strips. A 1 1b (454 gram) portion of these beef strips were placed on a plastic tray covered with sterile aluminum foil. This sample was then inoculated by spraying with the *E. coli* 11229 solution inoculum onto beef strips with the spray bottle. The tray was held at a slight angle and sprayed with the inoculum to cover the surface of the strips. For an average beef strip piece, about 5 squirts were required from the hand pumped spray bottle to cover the beef strip. Three squirts equal about 1 mL of solution. Each tray of beef strips had 15 squirts of inoculum applied. Inoculated meat trays were then placed in the cooler(~5°C) for 30 minutes to allow bacteria attachment. In the same manner a sample was treated with the *E. coli 0157:H7* cocktail solution inoculum for testing.

Antimicrobial Formulation preparation

Concentrate 30 of antimicrobial lipid was made by adding each components list in the Table 19 below into a glass container. The container was heated on a hot plate (50-80°C), during heating, the solution was constantly stirred, The solution was mixed until a homogenous transparent single-phase liquid resulted. This concentrate was used to treat the beef samples. In the same manner concentrate 31 was made for testing on beef strips.

Table 19 Concentrate Formulations

| | Formulation 30 | Formulation 31 |
|---|---|---|
| PGMC8 | 49.0 | 96.0 |
| Pationic 122A | 25.0 | |
| DOSS | 6.0 | 4.0 |
| Pluronic L44 | 5.0 | |
| Benzoic Acid | 15.0 | |

The concentrates were diluted with deionized (DI) water to give the compositions listed in Table 20 which were used to treat the beef strips. Each diluted solution was stirred with a magnet using the Fisher Thermix at a setting of 9 for at least 5 min before use.

Table 20

| Formulation (wt%) | 32 | 33 | 34 | 35 |
|---|---|---|---|---|
| Concentrate 30 | 4 | | | |
| Concentrate 31 | | 4 | 4 | 6 |
| Malic Acid | | 2.8 | | 2 |
| Tartaric Acid | | | 2.8 | |
| Lactic Acid | 2.8 | | | |
| Water | 93.2 | 93.2 | 93.2 | 92 |

Treatment of beef trim with antimicrobial and further processing

Each inoculated beef trim strip was dipped into a formulation solution for 30 seconds and then suspended to let excess solution drip for another 30 seconds from the beef strips. Each formulation 32-35 was tested for its effect in the same manner. Treated beef strips were stored for 1 hour after solution treatment in a cooler (5°C). The beef strips were then coarse ground using a Grinder (Berkel, La Porte, IN) with a 1/2" plate (US Edge 12 x ½) and then fine ground using 1/4" plate (DC 12 x ¼). The final ground beef meat samples were then stored in the cooler (5°C) until they were tested.

The ground beef samples were tested at various times for bacteria count .At each time point samples were run in triplicate for each treatment. A 25 gram portion was weighed and placed into a filtered Stomacher Bag (3M product) with 225 milliliters (mL) of Letheen Broth (VWR Scientific, Batavia, IL) and stomached for 30 seconds. Serial ten-fold sequential dilutions were made with Letheen broth. Samples were plated on PETRIFILM *Enterobacteriaceae* count plate (EB) (available from 3M, St. Paul, MN) PETRIFILM plates were incubated for 24 +/- 2 hours at 35°C and counted as recommended on the package insert. Plates with counts that were within the counting range of the plates (15-100 CFU per PETRIFILM EB plate) were used for analysis.

The results were converted to log 10 and the replicates averaged. The results of the treated meat samples were subtracted from the results of the analogous untreated meat samples to determine the log reduction of the treatment. Table 21 and 22 shows the log reduction of *E.coli* 11229 and the *E.coli* 0157:H7 cocktail.

Table 21.Log reduction of *E.coli* 11229

| Formulation | 32 | 33 | 34 | 35 |
|---|---|---|---|---|
| Day 1 | 1.636 | 2.354 | 1.874 | 1.911 |

(continued)

| Formulation | 32 | 33 | 34 | 35 |
|---|---|---|---|---|
| Day4 | | 3.283 | 2.676 | |
| Day6 | | 3.226 | 2.893 | |
| Day 7 | 1.586 | | | 2.189 |

Table 22.Log reduction of *E.coli* 0157:H7 cocktail

| Formulation | | |
|---|---|---|
| Day 1 | 0.586 | 1.426 |
| Day 7 | 1.390 | 2.503 |

## Claims

**1.** An antimicrobial composition, comprising:

Propylene glycol (C7-C14) fatty acid ester in a concentration high than any other individual component; and an enhancer, wherein the enhancer is an alpha-hydroxy acid, a beta-hydroxy acid, a carboxylic acid, a chelating agent, a phenolic compound, a (C1-C10) monohydroxy alcohol, bacteriocins, antimicrobial enzymes, iron-binding proteins and derivatives thereof, siderophores, sugars, or sugar alcohols;
wherein the propylene glycol (C7-C14) fatty acid ester comprises monoester in an amount greater than 60%.

**2.** The composition of claim 1, wherein the combination of the monoester and enhancer maintains stable activity.

**3.** The composition of claim 1, wherein the composition is stable at or above 4°C.

**4.** The composition of claim 1, wherein the concentration of propylene glycol ester remains substantially constant.

**5.** The composition of claim 1, wherein said fatty acid monoester is proprylene glycol monolaurate, propylene glycol monocaprylate, propylene glycol monocaprate, or combinations thereof.

**6.** The antimicrobial composition of claim 1, further comprising a surfactant.

**7.** The antimicrobial composition of claim 6, wherein the surfactant to ester ratio is 1:1 or less.

**8.** The composition of claim 1, further comprising a C8 to C14 fatty acid glycerol monoester.

**9.** An antimicrobial kit, comprising:

a first container with propylene glycol (C7-C14) fatty acid ester in a concentration higher than any other individual component comprising propylene glycol (C7-C14) fatty acid monoester in an amount grater than 60%;
a surfactant; and
a second container comprising an enhancer;
wherein the enhancer is an alpha-hydroxy acid, a beta-hydroxy acod, a carboxylic acid, a chelating agent, a phenolic compound, a (C1-C10) monohydroxy alcohol, bateriocins, antimicrobial enzymes, iron-binding proteins and derivatives thereof, siderophores, sugars, or sugar alcohols.

**10.** The antimicrobial kit of claim 9, wherein the combination of the ester and the enhancer maintains stable activity.

## Patentansprüche

**1.** Antimikrobielle Zusammensetzung, umfassend:

Propylenglykol-(C7-C14)-Fettsäureester in einer Konzentration über der Konzentration von einer beliebigen sonstigen Einzelkomponente; und
ein Verstärkungsmittel, wobei es sich bei dem Verstärkungsmittel um eine alpha-Hydroxysäure, eine beta-Hydroxysäure, eine Carbonsäure, ein Chelatisierungsmittel, eine phenolische Verbindung, einen (C1-C10)-Monohydroxyalkohol, Bacteriocine, antimikrobielle Enzyme, eisenbindende Proteine und Derivate davon, Siderophore, Zucker oder Zuckeralkohole handelt;
wobei der Propylenglykol-(C7-C14)-Fettsäureester Monoester in einer Menge von mehr als 60% umfasst.

2. Zusammensetzung nach Anspruch 1, wobei die Kombination des Monoesters und des Verstärkungsmittels eine stabile Aktivität aufrechterhält.

3. Zusammensetzung nach Anspruch 1, wobei die Zusammensetzung bei oder oberhalb 4°C stabil ist.

4. Zusammensetzung nach Anspruch 1, wobei die Propylenglykolesterzusammensetzung im Wesentlichen konstant bleibt.

5. Zusammensetzung nach Anspruch 1, wobei es sich bei dem Fettsäuremonoester um Propylenglykolmonolaurat, Propylenglykolmonocaprylat, Propylenglykolmonocaprat oder Kombinationen davon handelt.

6. Antimikrobielle Zusammensetzung nach Anspruch 1, die weiterhin ein Tensid umfasst.

7. Antimikrobielle Zusammensetzung nach Anspruch 6, wobei das Verhältnis zwischen Tensid zu Ester 1:1 oder weniger beträgt.

8. Zusammensetzung nach Anspruch 1, die weiterhin einen C8- bis C14-Fettsäureglycerolmonoester umfasst.

9. Antimikrobielles Kit, das Folgendes umfasst:

ein erstes Behältnis mit einem Propylenglykol-(C7-C14)-Fettsäureester in einer Konzentration über derjenigen einer beliebigen anderen Einzelkomponente,
umfassend Propylenglykol-(C7-C14)-Fettsäuremonoester in einer Menge von über 60%;
ein Tensid; und
ein zweites Behältnis, umfassend ein Verstärkungsmittel;
wobei es sich bei dem Verstärkungsmittel um eine alpha-Hydroxysäure, eine beta-Hydroxysäure, eine Carbonsäure, ein Chelatisierungsmittel, eine phenolische Verbindung, einen (C1-C10)-Monohydroxyalkohol, Bacteriocine, antimikrobielle Enzyme, eisenbindende Proteine und Derivate davon, Siderophore, Zucker oder Zuckeralkohole handelt.

10. Antimikrobielles Kit nach Anspruch 9, wobei die Kombination des Esters und des Verstärkungsmittels eine stabile Aktivität aufrechterhält.

**Revendications**

1. Composition antimicrobienne, comprenant :

un ester d'acide gras en C7-C14 de propylènegycol à une concentration supérieure à celle de tout autre composant individuel, et
un adjuvant, l'adjuvant étant un alpha-hydroxyacide, un bêta-hydroxyacide, un acide carboxylique, un agent chélateur, un composé phénolique, un monohydroxyalcool en C1-C10, des bactériocines, des enzymes antimicrobiennes,
des protéines de liaison de fer et des dérivés de celles-ci, des sidérophores, des glucides ou des polyols ;
l'ester d'acide gras en C7-C14 de propylènegycol comprenant un monoester en une quantité supérieure à 60 %.

2. Composition de la revendication 1, dans laquelle la combinaison du monoester et de l'adjuvant maintient une activité stable.

3. Composition de la revendication 1, dans laquelle la composition est stable à ou au-dessus de 4 °C.

**4.** Composition de la revendication 1, dans laquelle la concentration d'ester de propylèneglycol reste sensiblement constante.

**5.** Composition de la revendication 1, dans laquelle le monoester d'acide gras est le monolaurate de propylèneglycol, le monocaprylate de propylèneglycol, le monocaprate de propylèneglycol, ou des combinaisons de ceux-ci.

**6.** Composition antimicrobienne de la revendication 1, comprenant en outre un tensioactif.

**7.** Composition antimicrobienne de la revendication 6, dans laquelle le rapport du tensioactif à l'ester est de 1:1 ou moins.

**8.** Composition de la revendication 1, comprenant en outre un monoester de glycérol d'acide gras en C8 à C14.

**9.** Kit antimicrobien, comprenant :

un premier récipient avec un ester d'acide gras en C7-C14 de propylènegycol à une concentration supérieure à celle de tout autre composant individuel comprenant un monoester d'acide gras de propylèneglycol en C7-C14 en une quantité supérieure à 60%;
un tensioactif ; et
un deuxième récipient comprenant un adjuvant ;
l'adjuvant étant un alpha-hydroxyacide, un bêta-hydroxyacide, un acide carboxylique, un agent chélateur, un composé phénolique, un monohydroxyalcool en C1-C10, des bactériocines, des enzymes antimicrobiennes, des protéines de liaison de fer et des dérivés de celles-ci, des sidérophores, des glucides ou des polyols.

**10.** Kit antimicrobien de la revendication 9, dans lequel la combinaison de l'ester et de l'adjuvant maintient une activité stable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5460833 A
- US 5490992 A
- WO 200143549 A
- US 57254900 A
- US 4485029 A
- WO 9507616 A
- WO 0143549 A2
- US 4067997 A
- EP 0876768 A
- US 10659571 B
- US 96651101 A
- US 5665776 A, Yu
- US 20060051384 A
- US 20060052452 A
- US 20060051385 A
- US 5862949 A
- US 6045254 A
- US 6089389 A


### Non-patent literature cited in the description


- **Kabara.** *J. of Food Protection,* 1981, vol. 44, 633-647
- **Kabara.** *J. of Food Safety,* 1982, vol. 4, 13-25